(19) Europäisches Patentamt
European Patent Office
Office européen des brevets

(11) EP 0 638 122 B1

(12) EUROPEAN PATENT SPECIFICATION

(45) Date of publication and mention
of the grant of the patent:
**10.12.1997 Bulletin 1997/50**

(21) Application number: **93911702.4**

(22) Date of filing: **28.04.1993**

(51) Int Cl.6: **C12N 15/40**, C12N 7/00,
C07K 14/18, C12N 15/62,
A61K 39/12, C12P 21/00,
G01N 33/50

(86) International application number:
**PCT/CA93/00182**

(87) International publication number:
**WO 93/22440 (11.11.1993 Gazette 1993/27)**

(54) **CDNA SEQUENCE OF DENGUE VIRUS SEROTYPE 1 (SINGAPORE STRAIN)**

CDNS SEQUENZ DES DENGUE VIRUS SEROTYPE 1 (SINGAPUR STAMM)

SEQUENCE DE L'ADN COMPLEMENTAIRE DU SEROTYPE 1 DU VIRUS DE LA DENGUE
(SOUCHE SINGAPOUR)

(84) Designated Contracting States:
**AT BE CH DE DK ES FR GB GR IE IT LI LU NL PT
SE**

(30) Priority: **29.04.1992 GB 9209243**

(43) Date of publication of application:
**15.02.1995 Bulletin 1995/07**

(73) Proprietor: **NATIONAL UNIVERSITY OF
SINGAPORE
Singapore 119260 (SG)**

(72) Inventors:
• **TAN, Yin-Hwee**
**Singapore 0511 (SG)**
• **FU, Jianlin**
**Singapore 0511 (SG)**
• **TAN, Boon-Huan**
**Singapore 0511 (SG)**
• **YAP, Eu-Hian**
**Singapore 0511 (SG)**
• **CHAN, Yow-Cheong**
**Singapore 0511 (SG)**

(74) Representative: **Woods, Geoffrey Corlett
J.A. KEMP & CO.
14 South Square
Gray's Inn
London WC1R 5LX (GB)**

(56) References cited:
• **VIROLOGY vol. 188, no. 2, 1992, pages 953 - 958
FU,J. ET AL. 'Full-Length cDNA Sequence of
Dengue Type 1 Virus (Singapore Strain S275/90)'**
• **VIROLOGY vol. 174, no. 2, February 1990, pages
479 - 493 RICO-HESSE ,R. 'Molecular Evolution
and Distribution of Dengue Viruses Type 1 and
2 in Nature'**
• **VACCINES 80 1988, COLD SPRING HARBOR
pages 393 - 399 LAL, C.-J. ET AL. 'Cloning
Full-Length DNA Sequences of the Dengue Virus
Genome for use in Elucidating Pathogenesis and
Development of Immunoprophylaxis'**

SEQUENCE LISTING

(1) GENERAL INFORMATION:

    (i) APPLICANT:

        (A) NAME: National University of Singapore
        (B) STREET: 10 Kent Ridge Crescent
        (C) CITY: Singapore
        (E) COUNTRY: Singapore
        (F) POSTAL CODE (ZIP): 119260

    (ii) TITLE OF INVENTION: Dengue Virus

    (iii) NUMBER OF SEQUENCES: 12

    (iv) COMPUTER READABLE FORM:

        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25 (EPO)

    (v) CURRENT APPLICATION DATA:
        APPLICATION NUMBER:

(2) INFORMATION FOR SEQ ID NO:1:

    (i) SEQUENCE CHARACTERISTICS:

        (A) LENGTH: 10718 base pairs
        (B) TYPE: nucleic acid
        (C) STRANDEDNESS: single
        (D) TOPOLOGY: linear

    (ii) MOLECULE TYPE: cDNA corresponding to RNA (genomic)

    (iii) HYPOTHETICAL: NO

    (iv) ANTI-SENSE: NO

    (vi) ORIGINAL SOURCE:

        (A) ORGANISM: Dengue Fever Virus Type 1
        (B) STRAIN: S275/90

    (ix) FEATURE:

        (A) NAME/KEY: CDS
        (B) LOCATION: 81..10268

    (xi) SEQUENCE DESCRIPTION: SEQ ID NO:1:


```
GTGGACCGCA AAGAACAGTT TCGAATCGGA AGCTTGCTTA ACGTAGTTCT AACAGTTTTT          60

TATTAGAGAG CAGATCTCTG ATG AAC AAC CAA CGA AAA AAG ACG GCT CGA              110
                       Met Asn Asn Gln Arg Lys Lys Thr Ala Arg
                        1               5                   10

CCG TCT TTC AAT ATG CTG AAA CGC GCG AGA AAC CGC GTG TCA ACT GGT            158
Pro Ser Phe Asn Met Leu Lys Arg Ala Arg Asn Arg Val Ser Thr Gly
                15                  20                  25

TCA CAG TTG GCG AAG AGA TTC TCA AAA GGA TTG CTT TCA GGC CAA GGA            206
Ser Gln Leu Ala Lys Arg Phe Ser Lys Gly Leu Leu Ser Gly Gln Gly
                30                  35                  40
```

```
CCC ATG AAA TTG GTG ATG GCT TTC ATA GCA TTC CTA AGA TTT CTA GCC          254
Pro Met Lys Leu Val Met Ala Phe Ile Ala Phe Leu Arg Phe Leu Ala
        45              50              55

ATA CCC CCA ACA GCA GGA ATT TTG GCT AGA TGG GGC TCA TTC AAG AAG          302
Ile Pro Pro Thr Ala Gly Ile Leu Ala Arg Trp Gly Ser Phe Lys Lys
        60              65              70

AAT GGA GCG ATC AAA GTG CTA CGG GGT TTC AAG AAA GAA ATC TCA AAC          350
Asn Gly Ala Ile Lys Val Leu Arg Gly Phe Lys Lys Glu Ile Ser Asn
75              80              85              90

ATG TTG AAC ATA ATG AAT AGA AGG AAA AGA TCT GTG ACC ATG CTC CTC          398
Met Leu Asn Ile Met Asn Arg Arg Lys Arg Ser Val Thr Met Leu Leu
                95              100             105

ATG CTG CTG CCC ACA GCC TTG GCG TTC CAT TTG ACT ACA CGA GGG GGA          446
Met Leu Leu Pro Thr Ala Leu Ala Phe His Leu Thr Thr Arg Gly Gly
            110             115             120

GAG CCA CAC ATG ATA GTT AGC AAG CAG GAA AGA GAA AAG TCA CTC TTG          494
Glu Pro His Met Ile Val Ser Lys Gln Glu Arg Glu Lys Ser Leu Leu
            125             130             135

TTT AAG ACC TCT GTA GGT GTC AAC ATG TGC ACC CTT ATA GCG ATG GAT          542
Phe Lys Thr Ser Val Gly Val Asn Met Cys Thr Leu Ile Ala Met Asp
        140             145             150

TTG GGA GAG TTA TGT GAG GAC ACA ATG ACT TAC AAA TGC CCT CGA ATT          590
Leu Gly Glu Leu Cys Glu Asp Thr Met Thr Tyr Lys Cys Pro Arg Ile
155             160             165             170

ACT GAG GCG GAA CCA GAT GAC GTT GAT TGT TGG TGC AAT GCT ACA GAC          638
Thr Glu Ala Glu Pro Asp Asp Val Asp Cys Trp Cys Asn Ala Thr Asp
            175             180             185

ACA TGG GTG ACC TAT GGA ACA TGT TCC CAA ACT GGC GAG CAC CGA CGG          686
Thr Trp Val Thr Tyr Gly Thr Cys Ser Gln Thr Gly Glu His Arg Arg
            190             195             200

GAC AAA CGT TCC GTC GCA CTG GCC CCA CAC GTG GGA CTT GGT CTA GAA          734
Asp Lys Arg Ser Val Ala Leu Ala Pro His Val Gly Leu Gly Leu Glu
        205             210             215

ACA AGA ACC GAA ACG TGG ATG TCC TCT GAA GGC GCT TGG AAA CAA ATA          782
Thr Arg Thr Glu Thr Trp Met Ser Ser Glu Gly Ala Trp Lys Gln Ile
        220             225             230

CAA AGA GTG GAG ACT TGG GCT TTG CGA CAC CCA GGA TTC ACG GTG ATA          830
Gln Arg Val Glu Thr Trp Ala Leu Arg His Pro Gly Phe Thr Val Ile
235             240             245             250

GCC CTT TTT CTT GCA CAT GCC ATA GGA ACA TCC ATC ACT CAG AAA GGG          878
Ala Leu Phe Leu Ala His Ala Ile Gly Thr Ser Ile Thr Gln Lys Gly
            255             260             265

ATT ATT TTC ATT TTG TTA ATG CTA GTA ACA CCA TCC ATG GCC ATG CGA          926
Ile Ile Phe Ile Leu Leu Met Leu Val Thr Pro Ser Met Ala Met Arg
            270             275             280

TGC GTG GGA ATA GGC AGC AGG GAC TTC GTG GAA GGA CTA TCA GGA GCA          974
Cys Val Gly Ile Gly Ser Arg Asp Phe Val Glu Gly Leu Ser Gly Ala
            285             290             295
```

```
ACT TGG GTA GAC GTG GTA CTG GAA CAT GGA AGT TGC GTC ACC ACC ATG        1022
Thr Trp Val Asp Val Val Leu Glu His Gly Ser Cys Val Thr Thr Met
    300                 305                 310

GCA AAA GAC AAA CCA ACA TTG GAC ATT GAA CTC CTG AAA ACG GAG GTC        1070
Ala Lys Asp Lys Pro Thr Leu Asp Ile Glu Leu Leu Lys Thr Glu Val
315                 320                 325                 330

ACG AAC CCT GCC GTC CTG CGC AAA CTG TGC ATT GAA GCT AAA ATA TCA        1118
Thr Asn Pro Ala Val Leu Arg Lys Leu Cys Ile Glu Ala Lys Ile Ser
                335                 340                 345

AAC ACC ACC ACC GAT TCA AGA TGT CCA ACA CAA GGA GAA GCT ACA CTG        1166
Asn Thr Thr Thr Asp Ser Arg Cys Pro Thr Gln Gly Glu Ala Thr Leu
                350                 355                 360

GTG GAA GAA CAA GAC GCG AAC TTT GTG TGT CGA CGA ACG TTC GTG GAC        1214
Val Glu Glu Gln Asp Ala Asn Phe Val Cys Arg Arg Thr Phe Val Asp
            365                 370                 375

AGA GGC TGG GGT AAT GGC TGC GGA CTA TTT GGA AAA GGA AGC CTA CTG        1262
Arg Gly Trp Gly Asn Gly Cys Gly Leu Phe Gly Lys Gly Ser Leu Leu
        380                 385                 390

ACG TGT GCT AAG TTC AAG TGT GTG ACA AAA CTA GAA GGA AAG ATA GTT        1310
Thr Cys Ala Lys Phe Lys Cys Val Thr Lys Leu Glu Gly Lys Ile Val
395                 400                 405                 410

CAA TAT GAA AAC TTA AAA TAT TCA GTG ATA GTC ACT GTC CAC ACT GGG        1358
Gln Tyr Glu Asn Leu Lys Tyr Ser Val Ile Val Thr Val His Thr Gly
                415                 420                 425

GAC CAG CAC CAG GTG GGA AAC GAG ACT ACA GAA CAT GGA ACA ATT GCA        1406
Asp Gln His Gln Val Gly Asn Glu Thr Thr Glu His Gly Thr Ile Ala
            430                 435                 440

ACC ATA ACA CCT CAA GCT CCT ACG TCG GAA ATA CAG CTG ACC GAC TAC        1454
Thr Ile Thr Pro Gln Ala Pro Thr Ser Glu Ile Gln Leu Thr Asp Tyr
        445                 450                 455

GGA GCC CTC ACA TTG GAC TGC TCA CCT AGA ACT GGG CTG GAC TTT AAT        1502
Gly Ala Leu Thr Leu Asp Cys Ser Pro Arg Thr Gly Leu Asp Phe Asn
        460                 465                 470

GAG ATG GTG CTA TTG ACA ATG AAA GAA AAA TCA TGG CTT GTT CAC AAA        1550
Glu Met Val Leu Leu Thr Met Lys Glu Lys Ser Trp Leu Val His Lys
475                 480                 485                 490

CAA TGG TTT CTA GAC TTA CCA CTG CCT TGG ACT TCG GGG GCT TCA ACA        1598
Gln Trp Phe Leu Asp Leu Pro Leu Pro Trp Thr Ser Gly Ala Ser Thr
                495                 500                 505

TCC CAA GAG ACT TGG AAC AGA CAA GAT TTG CTG GTC ACA TTC AAG ACA        1646
Ser Gln Glu Thr Trp Asn Arg Gln Asp Leu Leu Val Thr Phe Lys Thr
                510                 515                 520

GCT CAT GCA AAG AAG CAG GAA GTA GTC GTA CTG GGA TCA CAG GAA GGA        1694
Ala His Ala Lys Lys Gln Glu Val Val Val Leu Gly Ser Gln Glu Gly
        525                 530                 535

GCA ATG CAC ACT GCG TTG ACT GGG GCG ACA GAA ATC CAA ACG TCT GGA        1742
Ala Met His Thr Ala Leu Thr Gly Ala Thr Glu Ile Gln Thr Ser Gly
        540                 545                 550
```

EP 0 638 122 B1

```
ACG ACA ACA ATT TTT GCA GGA CAC CTG AAA TGT AGA CTA AAA ATG GAC        1790
Thr Thr Thr Ile Phe Ala Gly His Leu Lys Cys Arg Leu Lys Met Asp
555             560             565             570

AAA CTG ACT CTA AAA GGG ATG TCA TAT GTG ATG TGC ACA GGC TCA TTT        1838
Lys Leu Thr Leu Lys Gly Met Ser Tyr Val Met Cys Thr Gly Ser Phe
                575             580             585

AAG CTA GAG AAG GAA GTG GCT GAG ACC CAG CAT GGA ACT GTT TTA GTG        1886
Lys Leu Glu Lys Glu Val Ala Glu Thr Gln His Gly Thr Val Leu Val
                590             595             600

CAG GTT AAA TAC GAA GGA ACA GAT GCA CCA TGC AAG ATC CCC TTT TCG        1934
Gln Val Lys Tyr Glu Gly Thr Asp Ala Pro Cys Lys Ile Pro Phe Ser
            605             610             615

ACC CAA GAT GAG AAA GGA GTG ACC CAG AAT AGA TTG ATA ACA GCC AAT        1982
Thr Gln Asp Glu Lys Gly Val Thr Gln Asn Arg Leu Ile Thr Ala Asn
        620             625             630

CCT ATA GTT ACT GAC AAA GAA AAA CCA GTC AAC ATT GAG ACA GAA CCA        2030
Pro Ile Val Thr Asp Lys Glu Lys Pro Val Asn Ile Glu Thr Glu Pro
635             640             645             650

CCT TTT GGT GAG AGC TAC ATC GTG GTA GGG GCA GGT GAA AAA GCT TTG        2078
Pro Phe Gly Glu Ser Tyr Ile Val Val Gly Ala Gly Glu Lys Ala Leu
                655             660             665

AAA CAA TGC TGG TTC AAG AAA GGA AGC AGC ATA GGG AAA ATG TTC GAA        2126
Lys Gln Cys Trp Phe Lys Lys Gly Ser Ser Ile Gly Lys Met Phe Glu
            670             675             680

GCA ACC GCC CGA GGA GCA CGA AGG ATG GCT ATC CTG GGA GAC ACC GCA        2174
Ala Thr Ala Arg Gly Ala Arg Arg Met Ala Ile Leu Gly Asp Thr Ala
            685             690             695

TGG GAC TTC GGT TCT ATA GGA GGA GTG TTC ACG TCT GTG GGA AAA TTA        2222
Trp Asp Phe Gly Ser Ile Gly Gly Val Phe Thr Ser Val Gly Lys Leu
            700             705             710

GTG CAT CAG GTT TTT GGA ACC GCA TAT GGG GTT CTG TTC AGC GGT GTT        2270
Val His Gln Val Phe Gly Thr Ala Tyr Gly Val Leu Phe Ser Gly Val
715             720             725             730

TCT TGG ACC ATG AAA ATA GGA ATA GGG ATT CTG CTG ACA TGG TTG GGA        2318
Ser Trp Thr Met Lys Ile Gly Ile Gly Ile Leu Leu Thr Trp Leu Gly
                735             740             745

TTA AAT TCA AGG AGC ACG TCA CTT TCG ATG ACG TGC ATT GCA GTT GGC        2366
Leu Asn Ser Arg Ser Thr Ser Leu Ser Met Thr Cys Ile Ala Val Gly
            750             755             760

ATG GTC ACA CTG TAC CTA GGA GTC ATG GTT CAA GCG GAC TCG GGA TGT        2414
Met Val Thr Leu Tyr Leu Gly Val Met Val Gln Ala Asp Ser Gly Cys
            765             770             775

GTA ATC AAC TGG AAG GGC AGA GAA CTC AAA TGT GGA AGT GGC ATT TTT        2462
Val Ile Asn Trp Lys Gly Arg Glu Leu Lys Cys Gly Ser Gly Ile Phe
        780             785             790

GTC ACT AAT GAA GTC CAC ACT TGG ACA GAG CAA TAC AAA TTT CAA GCT        2510
Val Thr Asn Glu Val His Thr Trp Thr Glu Gln Tyr Lys Phe Gln Ala
795             800             805             810
```

14

```
GAC TCC CCA AAA AGA CTA TCA GCA GCC ATC GGA AAG GCA TGG GAG GAG        2558
Asp Ser Pro Lys Arg Leu Ser Ala Ala Ile Gly Lys Ala Trp Glu Glu
            815                 820                 825

GGT GTG TGT GGA ATT CGA TCA GCC ACT CGT CTC GAG AAC ATC ATG TGG        2606
Gly Val Cys Gly Ile Arg Ser Ala Thr Arg Leu Glu Asn Ile Met Trp
            830                 835                 840

AAG CAA ATA TCA AAT GAA CTG AAC CAC ATC TTA CTT GAA AAT GAC ATG        2654
Lys Gln Ile Ser Asn Glu Leu Asn His Ile Leu Leu Glu Asn Asp Met
            845                 850                 855

AAA TTC ACA GTG GTT GTA GGA GAT GTT GTT GGG ATC TTG GCC CAA GGG        2702
Lys Phe Thr Val Val Val Gly Asp Val Val Gly Ile Leu Ala Gln Gly
            860                 865                 870

AAA AAA ATG ATT AGA CCA CAA CCC ATG GAA CAC AAA TAC TCA TGG AAA        2750
Lys Lys Met Ile Arg Pro Gln Pro Met Glu His Lys Tyr Ser Trp Lys
875                 880                 885                 890

AGC TGG GGA AAA GCC AAA ATC ATA GGA GCA GAC ATA CAG AAC ACC ACC        2798
Ser Trp Gly Lys Ala Lys Ile Ile Gly Ala Asp Ile Gln Asn Thr Thr
            895                 900                 905

TTC ATC ATT GAC GGC CCA GAT ACT CCA GAA TGT CCT GAT GAC CAA AGA        2846
Phe Ile Ile Asp Gly Pro Asp Thr Pro Glu Cys Pro Asp Asp Gln Arg
            910                 915                 920

GCA TGG AAC ATT TGG GAA GTT GAG GAC TAT GGG TTC GGA ATT TTC ACG        2894
Ala Trp Asn Ile Trp Glu Val Glu Asp Tyr Gly Phe Gly Ile Phe Thr
            925                 930                 935

ACA AAC ATA TGG TTG AAA TTG CGT GAC TCC TAC ACC CAA ATG TGT GAC        2942
Thr Asn Ile Trp Leu Lys Leu Arg Asp Ser Tyr Thr Gln Met Cys Asp
            940                 945                 950

CAC CGG CTA ATG TCA GCT GCC ATC AAG GAC AGC AAG GCA GTC CAT GCT        2990
His Arg Leu Met Ser Ala Ala Ile Lys Asp Ser Lys Ala Val His Ala
955                 960                 965                 970

GAT ATG GGG TAC TGG ATA GAA AGT GAA AAG AAC GAG ACC TGG AAG CTG        3038
Asp Met Gly Tyr Trp Ile Glu Ser Glu Lys Asn Glu Thr Trp Lys Leu
            975                 980                 985

GCA AGA GCC TCT TTC ATA GAA GTT AAA ACA TGT GTC TGG CCA AAA TCC        3086
Ala Arg Ala Ser Phe Ile Glu Val Lys Thr Cys Val Trp Pro Lys Ser
            990                 995                 1000

CAC ACT CTA TGG AGC AAT GGA GTT CTG GAA AGT GAA ATG ATA ATT CCA        3134
His Thr Leu Trp Ser Asn Gly Val Leu Glu Ser Glu Met Ile Ile Pro
            1005                1010                1015

AAG ATC TAT GGA GGA CCA ATA TCT CAG CAC AAC TAC AGA CCA GGA TAT        3182
Lys Ile Tyr Gly Gly Pro Ile Ser Gln His Asn Tyr Arg Pro Gly Tyr
            1020                1025                1030

TTC ACA CAA ACG GCA GGG CCA TGG CAC CTA GGC AAG TTG GAA CTG GAT        3230
Phe Thr Gln Thr Ala Gly Pro Trp His Leu Gly Lys Leu Glu Leu Asp
1035                1040                1045                1050

TTT GAT TTG TGT GAG GGT ACC ACA GTT GTT GTG GAT GAA CAT TGT GGA        3278
Phe Asp Leu Cys Glu Gly Thr Thr Val Val Val Asp Glu His Cys Gly
            1055                1060                1065
```

```
AAT CGA GGT CCA TCT CTT AGA ACC ACA ACA GTC ACA GGA AAG ATA ATT      3326
Asn Arg Gly Pro Ser Leu Arg Thr Thr Thr Val Thr Gly Lys Ile Ile
        1070            1075                1080

CAT GAA TGG TGT TGC AGA TCT TGT ACG CTA CCA CCC TTA CGT TTC AAA      3374
His Glu Trp Cys Cys Arg Ser Cys Thr Leu Pro Pro Leu Arg Phe Lys
        1085            1090                1095

GGA GAA GAT GGA TGT TGG TAC GGT ATG GAA ATC AGA CCA GTC AAG GAA      3422
Gly Glu Asp Gly Cys Trp Tyr Gly Met Glu Ile Arg Pro Val Lys Glu
    1100            1105                1110

AAG GAA GAG AAT CTA GTC AAA TCA ATG GTC TCT GCA GGG TCA GGG GAA      3470
Lys Glu Glu Asn Leu Val Lys Ser Met Val Ser Ala Gly Ser Gly Glu
1115                1120                1125                1130

GTG GAC AGC TTT TCA CTA GGA CTG CTA TGC ATA TCA ATA ATG ATC GAA      3518
Val Asp Ser Phe Ser Leu Gly Leu Leu Cys Ile Ser Ile Met Ile Glu
            1135                1140                1145

GAG GTG ATG AGA TCC AGA TGG AGC AGA AAA ATG CTG ATG ACT GGA ACA      3566
Glu Val Met Arg Ser Arg Trp Ser Arg Lys Met Leu Met Thr Gly Thr
            1150                1155                1160

CTG GCT GTG TTC CTC CTT CTC ATA ATG GGA CAA TTG ACA TGG AAT GAT      3614
Leu Ala Val Phe Leu Leu Leu Ile Met Gly Gln Leu Thr Trp Asn Asp
        1165                1170                1175

CTG ATC AGG TTA TGC ATC ATG GTT GGA GCC AAT GCT TCA GAC AGG ATG      3662
Leu Ile Arg Leu Cys Ile Met Val Gly Ala Asn Ala Ser Asp Arg Met
        1180                1185                1190

GGG ATG GGA ACA ACG TAC CTA GCT CTG ATG GCC ACT TTT AAA ATG AGA      3710
Gly Met Gly Thr Thr Tyr Leu Ala Leu Met Ala Thr Phe Lys Met Arg
1195                1200                1205                1210

CCA ATG TTT GCT GTC GGG CTG TTG TTC CGC AGA CTA ACA TCT AGA GAA      3758
Pro Met Phe Ala Val Gly Leu Leu Phe Arg Arg Leu Thr Ser Arg Glu
            1215                1220                1225

GTT CTT CTT CTT ACA ATT GGA TTG AGT CTA GTG GCA TCT GTG GAG TTA      3806
Val Leu Leu Leu Thr Ile Gly Leu Ser Leu Val Ala Ser Val Glu Leu
        1230                1235                1240

CCA AAT TCC CTG GAG GAG CTG GGG GAT GGA CTT GCA ATG GGC ATT ATG      3854
Pro Asn Ser Leu Glu Glu Leu Gly Asp Gly Leu Ala Met Gly Ile Met
        1245                1250                1255

ATT TTA AAA TTA TTG ACT GAC TTT CAG TCA CAT CAG CTG TGG GCT ACC      3902
Ile Leu Lys Leu Leu Thr Asp Phe Gln Ser His Gln Leu Trp Ala Thr
        1260                1265                1270

TTG CTG TCC TTG ACA TTT GTC AAA ACA ACG TTT TCC TTG CAC TAT GCA      3950
Leu Leu Ser Leu Thr Phe Val Lys Thr Thr Phe Ser Leu His Tyr Ala
1275                1280                1285                1290

TGG AAG ACA ATG GCT ATG GTA CTG TCA ATT GTA TCT CTC TTC CCC TTA      3998
Trp Lys Thr Met Ala Met Val Leu Ser Ile Val Ser Leu Phe Pro Leu
            1295                1300                1305

TGC CTG TCC ACG ACC TCC CAA AAA ACA ACA TGG CTT CCG GTG CTA TTG      4046
Cys Leu Ser Thr Thr Ser Gln Lys Thr Thr Trp Leu Pro Val Leu Leu
        1310                1315                1320
```

```
GGA TCT CTT GGA TGC AAA CCA CTA ACC ATG TTT CTC ATA GCA GAA AAC        4094
Gly Ser Leu Gly Cys Lys Pro Leu Thr Met Phe Leu Ile Ala Glu Asn
        1325            1330            1335

AAA ATC TGG GGA AGG AAA AGT TGG CCC CTC AAT GAA GGA ATC ATG GCT        4142
Lys Ile Trp Gly Arg Lys Ser Trp Pro Leu Asn Glu Gly Ile Met Ala
        1340            1345            1350

GTT GGA ATA GTC AGC ATC CTA CTA AGT TCA CTC CTC AAA AAT GAT GTG        4190
Val Gly Ile Val Ser Ile Leu Leu Ser Ser Leu Leu Lys Asn Asp Val
1355            1360            1365            1370

CCG CTA GCT GGG CCA CTA ATA GCT GGA GGC ATG CTA ATA GCA TGT TAC        4238
Pro Leu Ala Gly Pro Leu Ile Ala Gly Gly Met Leu Ile Ala Cys Tyr
                1375            1380            1385

GTT ATA TCT GGA AGC TCA GCC GAC TTA TCA CTA GAG AAA GCG GCT GAG        4286
Val Ile Ser Gly Ser Ser Ala Asp Leu Ser Leu Glu Lys Ala Ala Glu
            1390            1395            1400

GTC TCC TGG GAA GAA GAA GCA GAA CAC TCT GGT GCC TCA CAC AAT ATA        4334
Val Ser Trp Glu Glu Glu Ala Glu His Ser Gly Ala Ser His Asn Ile
            1405            1410            1415

TTA GTG GAG GTC CAA GAT GAT GGA ACC ATG AAG ATA AAA GAT GAA GAG        4382
Leu Val Glu Val Gln Asp Asp Gly Thr Met Lys Ile Lys Asp Glu Glu
        1420            1425            1430

AGA GAT GAC ACG CTA ACC ATT CTC CTT AAA GCA ACC CTG CTA GCA GTT        4430
Arg Asp Asp Thr Leu Thr Ile Leu Leu Lys Ala Thr Leu Leu Ala Val
1435            1440            1445            1450

TCA GGG GTG TAC CCA TTA TCA ATA CCA GCA ACC CTT TTT GTG TGG TAC        4478
Ser Gly Val Tyr Pro Leu Ser Ile Pro Ala Thr Leu Phe Val Trp Tyr
                1455            1460            1465

TTT TGG CAG AAA AAG AAA CAA AGA TCT GGA GTG TTA TGG GAC ACA CCT        4526
Phe Trp Gln Lys Lys Lys Gln Arg Ser Gly Val Leu Trp Asp Thr Pro
            1470            1475            1480

AGC CCT CCA GAA GTG GAA AGA GCA GTC CTT GAT GAT GGT ATC TAT AGA        4574
Ser Pro Pro Glu Val Glu Arg Ala Val Leu Asp Asp Gly Ile Tyr Arg
            1485            1490            1495

ATT ATG CAG AGA GGA CTG TTG GGC AGG TCC CAA GTA GGA GTG GGA GTT        4622
Ile Met Gln Arg Gly Leu Leu Gly Arg Ser Gln Val Gly Val Gly Val
        1500            1505            1510

TTC CAA GAC GGC GTG TTC CAC ACA ATG TGG CAC GTC ACC AGG GGA GCT        4670
Phe Gln Asp Gly Val Phe His Thr Met Trp His Val Thr Arg Gly Ala
1515            1520            1525            1530

GTC CTT ATG TAC CAA GGG AAG AGG CTG GAA CCA AGC TGG GCC AGT GTC        4718
Val Leu Met Tyr Gln Gly Lys Arg Leu Glu Pro Ser Trp Ala Ser Val
                1535            1540            1545

AAA AAA GAC TTG ATC TCA TAT GGA GGA GGT TGG AGG TTT CAA GGA TCC        4766
Lys Lys Asp Leu Ile Ser Tyr Gly Gly Gly Trp Arg Phe Gln Gly Ser
                1550            1555            1560

TGG AAC ACG GGA GAA GAA GTG CAG GTG ATT GCT GTT GAA CCA GGA AAA        4814
Trp Asn Thr Gly Glu Glu Val Gln Val Ile Ala Val Glu Pro Gly Lys
            1565            1570            1575
```

```
AAC CCC AAA AAT GTA CAG ACA GCG CCG GGT ACC TTC AAG ACC CCT GAA          4862
Asn Pro Lys Asn Val Gln Thr Ala Pro Gly Thr Phe Lys Thr Pro Glu
    1580                1585                1590

GGT GAA GTT GGA GCT ATT GCC CTA GAT TTT AAA CCC GGC ACA TCT GGA          4910
Gly Glu Val Gly Ala Ile Ala Leu Asp Phe Lys Pro Gly Thr Ser Gly
1595                1600                1605                1610

TCT CCC ATC GTG AAC AGA GAA GGA AAA ATA GTA GGT CTT TAT GGA AAT          4958
Ser Pro Ile Val Asn Arg Glu Gly Lys Ile Val Gly Leu Tyr Gly Asn
                1615                1620                1625

GGA GTA GTG ACA ACA AGT GGA ACC TAC GTC AGT GCC ATA GCC CAA GCC          5006
Gly Val Val Thr Thr Ser Gly Thr Tyr Val Ser Ala Ile Ala Gln Ala
                1630                1635                1640

AAA GCA TCA CAA GAA GGG CCC CTA CCA GAG ATT GAG GAC GAG GTG TTT          5054
Lys Ala Ser Gln Glu Gly Pro Leu Pro Glu Ile Glu Asp Glu Val Phe
                1645                1650                1655

AGG AAA AGA AAC TTA ACA ATA ATG GAC CTA CAT CCA GGA TCG GGG AAA          5102
Arg Lys Arg Asn Leu Thr Ile Met Asp Leu His Pro Gly Ser Gly Lys
    1660                1665                1670

ACA AGA AGA TAT CTT CCA GCC ATA GTC CGT GAG GCC ATA AGA AGG AAC          5150
Thr Arg Arg Tyr Leu Pro Ala Ile Val Arg Glu Ala Ile Arg Arg Asn
1675                1680                1685                1690

GTG CGC ACA CTA ATT TTG GCT CCC ACA AGG GTT GTC GCT TCC GAA ATG          5198
Val Arg Thr Leu Ile Leu Ala Pro Thr Arg Val Val Ala Ser Glu Met
                1695                1700                1705

GCA GAG GCG CTC AAG GGA ATG CCA ATA AGG TAC CAA ACA ACA GCA GTG          5246
Ala Glu Ala Leu Lys Gly Met Pro Ile Arg Tyr Gln Thr Thr Ala Val
                1710                1715                1720

AAG AGT GAA CAC ACA GGA AAA GAG ATA GTT GAC CTC ATG TGT CAC GCC          5294
Lys Ser Glu His Thr Gly Lys Glu Ile Val Asp Leu Met Cys His Ala
                1725                1730                1735

ACT TTC ACC ATG CGT CTC CTG TCT CCC GTG AGA GTT CCC AAT TAC AAC          5342
Thr Phe Thr Met Arg Leu Leu Ser Pro Val Arg Val Pro Asn Tyr Asn
    1740                1745                1750

ATG ATT ATC ATG GAT GAA GCA CAT TTT ACC GAT CCA GCC AGC ATA GCG          5390
Met Ile Ile Met Asp Glu Ala His Phe Thr Asp Pro Ala Ser Ile Ala
1755                1760                1765                1770

CGC AGA GGG TAC ATC TCA ACC CGA GTG GGC ATG GGT GAA GCA GCT GCG          5438
Arg Arg Gly Tyr Ile Ser Thr Arg Val Gly Met Gly Glu Ala Ala Ala
                1775                1780                1785

ATC TTC ATG ACA GCC ACT CCC CCA GGA TCG GTG GAG GCC TTT CCA CAG          5486
Ile Phe Met Thr Ala Thr Pro Pro Gly Ser Val Glu Ala Phe Pro Gln
                1790                1795                1800

AGC AAT GCA GTT ATC CAA GAT GAG GAA AGA GAC ATT CCT GAG AGA TCA          5534
Ser Asn Ala Val Ile Gln Asp Glu Glu Arg Asp Ile Pro Glu Arg Ser
                1805                1810                1815

TGG AAC TCA GGC TAT GAG TGG ATC ACT GAC TTC CCA GGT AAA ACA GTC          5582
Trp Asn Ser Gly Tyr Glu Trp Ile Thr Asp Phe Pro Gly Lys Thr Val
    1820                1825                1830
```

18

```
TGG TTT GTT CCA AGC ATC AAA TCA GGA AAT GAC ATT GCC AAC TGC TTA          5630
Trp Phe Val Pro Ser Ile Lys Ser Gly Asn Asp Ile Ala Asn Cys Leu
1835                1840                1845                1850

AGA AAG AAT GGG AAA CGG GTG ATT CAA TTG AGC AGG AAA ACC TTT GAT          5678
Arg Lys Asn Gly Lys Arg Val Ile Gln Leu Ser Arg Lys Thr Phe Asp
                1855                1860                1865

ACA GAG TAC CAA AAA ACA AAA AAC AAC GAC TGG GAC TAT GTC GTC ACA          5726
Thr Glu Tyr Gln Lys Thr Lys Asn Asn Asp Trp Asp Tyr Val Val Thr
                1870                1875                1880

ACA GAT ATC TCC GAA ATG GGA GCA AAC TTC CGA GCC GAC AGG GTG ATA          5774
Thr Asp Ile Ser Glu Met Gly Ala Asn Phe Arg Ala Asp Arg Val Ile
            1885                1890                1895

GAC CCA AGA CGG TGT CTG AAA CCG GTA ATA CTA AAA GAT GGT CCA GAG          5822
Asp Pro Arg Arg Cys Leu Lys Pro Val Ile Leu Lys Asp Gly Pro Glu
            1900                1905                1910

CGC GTC ATT CTA GCC GGA CCG ATG CCA GTG ACT GTG GCC AGT GCT GCC          5870
Arg Val Ile Leu Ala Gly Pro Met Pro Val Thr Val Ala Ser Ala Ala
1915                1920                1925                1930

CAG AGG AGA GGA AGA ATT GGA AGG AAC CAA AAC AAA GAA GGT GAT CAG          5918
Gln Arg Arg Gly Arg Ile Gly Arg Asn Gln Asn Lys Glu Gly Asp Gln
                1935                1940                1945

TAC GTT TAC ATG GGA CAG CCT TTA AAT AAT GAT GAG GAT CAC GCT CAT          5966
Tyr Val Tyr Met Gly Gln Pro Leu Asn Asn Asp Glu Asp His Ala His
                1950                1955                1960

TGG ACA GAA GCA AAA ATG CTC CTT GAC AAT ATA AAC ACA CCA GAA GGG          6014
Trp Thr Glu Ala Lys Met Leu Leu Asp Asn Ile Asn Thr Pro Glu Gly
                1965                1970                1975

ATC ATC CCA GCC CTC TTT GAG CCA GAG AGA GAA AAG AGT GCA GCA ATA          6062
Ile Ile Pro Ala Leu Phe Glu Pro Glu Arg Glu Lys Ser Ala Ala Ile
            1980                1985                1990

GAC GGG GAG TAC AGA CTG CGG GGA GAA GCA AGA AAA ACG TTT GTG GAG          6110
Asp Gly Glu Tyr Arg Leu Arg Gly Glu Ala Arg Lys Thr Phe Val Glu
1995                2000                2005                2010

CTC ATG AGA AGA GGA GAT CTA CCT GTC TGG CTA TCC TAC AAA GTT GCC          6158
Leu Met Arg Arg Gly Asp Leu Pro Val Trp Leu Ser Tyr Lys Val Ala
                2015                2020                2025

TCA GAA GGC TTC CAG TAC TCT GAC AGA AGA TGG TGC TTT GAC GGG GAA          6206
Ser Glu Gly Phe Gln Tyr Ser Asp Arg Arg Trp Cys Phe Asp Gly Glu
                2030                2035                2040

AGG AAC AAC CAG GTG TTG GAG GAG AAC ATG GAC GTG GAG ATG TGG ACA          6254
Arg Asn Asn Gln Val Leu Glu Glu Asn Met Asp Val Glu Met Trp Thr
                2045                2050                2055

AAA GAA GGA GAA CGA AAG AAA CTA CGA CCC CGC TGG CTG GAT GCC AGA          6302
Lys Glu Gly Glu Arg Lys Lys Leu Arg Pro Arg Trp Leu Asp Ala Arg
                2060                2065                2070

ACA TAC TCA GAC CCA CTG GCC CTG CGC GAG TTT AAA GAG TTT GCA GCA          6350
Thr Tyr Ser Asp Pro Leu Ala Leu Arg Glu Phe Lys Glu Phe Ala Ala
2075                2080                2085                2090
```

19

```
GGA AGA AGA AGT GTC TCA GGT GAT CTA ATA TTA GAA ATA GGG AAA CTT        6398
Gly Arg Arg Ser Val Ser Gly Asp Leu Ile Leu Glu Ile Gly Lys Leu
            2095                2100                2105

CCA CAA CAC TTG ACG CAA AGG GCC CAG AAT GCC TTG GAC AAC CTG GTT        6446
Pro Gln His Leu Thr Gln Arg Ala Gln Asn Ala Leu Asp Asn Leu Val
            2110                2115                2120

ATG TTG CAC AAC TCC GAA CAA GGA GGA AGA GCC TAC AGA CAT GCA ATG        6494
Met Leu His Asn Ser Glu Gln Gly Gly Arg Ala Tyr Arg His Ala Met
            2125                2130                2135

GAA GAA CTT CCA GAC ACC ATA GAA ACG TTG ATG CTC CTA GCT TTG ATA        6542
Glu Glu Leu Pro Asp Thr Ile Glu Thr Leu Met Leu Leu Ala Leu Ile
            2140                2145                2150

GCT GTG TTA ACT GGT GGA GTG ACG CTG TTC TTC CTA TCA GGA AAG GGC        6590
Ala Val Leu Thr Gly Gly Val Thr Leu Phe Phe Leu Ser Gly Lys Gly
2155                2160                2165                2170

CTA GGG AAA ACA TCT ATT GGC CTA CTC TGC GTG ATG GCT TCA AGC GTA        6638
Leu Gly Lys Thr Ser Ile Gly Leu Leu Cys Val Met Ala Ser Ser Val
            2175                2180                2185

CTG CTA TGG ATG GCC AGC GTG GAG CCT CAT TGG ATA GCG GCC TCC ATC        6686
Leu Leu Trp Met Ala Ser Val Glu Pro His Trp Ile Ala Ala Ser Ile
            2190                2195                2200

ATA CTA GAG TTT TTC CTG ATG GTG CTG CTT ATT CCA GAG CCA GAC AGA        6734
Ile Leu Glu Phe Phe Leu Met Val Leu Leu Ile Pro Glu Pro Asp Arg
            2205                2210                2215

CAG CGC ACT CCA CAG GAC AAC CAG TTA GCA TAT GTG GTG ATA GGT TTG        6782
Gln Arg Thr Pro Gln Asp Asn Gln Leu Ala Tyr Val Val Ile Gly Leu
            2220                2225                2230

TTA TTC ATG ATA CTC ACA GTG GCA GCC AAT GAG ATG GGA TTA TTG GAA        6830
Leu Phe Met Ile Leu Thr Val Ala Ala Asn Glu Met Gly Leu Leu Glu
2235                2240                2245                2250

ACC ACA AAG AAA GAC TTA GGG ATT GGC CAT GTA GCC GCC GAA AAC CAC        6878
Thr Thr Lys Lys Asp Leu Gly Ile Gly His Val Ala Ala Glu Asn His
            2255                2260                2265

CAC CAT GCT ACA ATG CTG GAC GTA GAC CTA CGT CCA GCT TCA GCC TGG        6926
His His Ala Thr Met Leu Asp Val Asp Leu Arg Pro Ala Ser Ala Trp
            2270                2275                2280

ACC CTC TAT GCA GTA GCC ACA ACA GTT ATC ACC CCC ATG ATG AGA CAC        6974
Thr Leu Tyr Ala Val Ala Thr Thr Val Ile Thr Pro Met Met Arg His
            2285                2290                2295

ACA ATT GAA AAT ACA ACG GCA AAT ATT TCC CTG ACA GCC ATT GCA AAC        7022
Thr Ile Glu Asn Thr Thr Ala Asn Ile Ser Leu Thr Ala Ile Ala Asn
            2300                2305                2310

CAG GCA GCT ATA TTG ATG GGA CTT GAT AAA GGA TGG CCA ATA TCG AAG        7070
Gln Ala Ala Ile Leu Met Gly Leu Asp Lys Gly Trp Pro Ile Ser Lys
2315                2320                2325                2330

ATG GAC ATA GGA GTT CCA CTT CTC GCC TTG GGG TGC TAT TCC CAG GTG        7118
Met Asp Ile Gly Val Pro Leu Leu Ala Leu Gly Cys Tyr Ser Gln Val
            2335                2340                2345
```

20

```
AAT CCA CTG ACG CTG ACA GCG GCG GTA TTG ATG CTA GTG GCT CAT TAC          7166
Asn Pro Leu Thr Leu Thr Ala Ala Val Leu Met Leu Val Ala His Tyr
        2350                2355                2360

GCC ATA ATT GGA CCT GGA CTG CAA GCA AAA GCG ACT AGA GAA GCT CAA          7214
Ala Ile Ile Gly Pro Gly Leu Gln Ala Lys Ala Thr Arg Glu Ala Gln
        2365                2370                2375

AAA AGG ACA GCG GCC GGA ATA ATG AAA AAT CCA ACC GTT GAT GGA ATT          7262
Lys Arg Thr Ala Ala Gly Ile Met Lys Asn Pro Thr Val Asp Gly Ile
        2380                2385                2390

GTT GCA ATA GAT TTG GAC CCT GTG GTT TAT GAT GCA AAA TTT GAG AAA          7310
Val Ala Ile Asp Leu Asp Pro Val Val Tyr Asp Ala Lys Phe Glu Lys
2395                2400                2405                2410

CAA CTA GGC CAA ATA ATG TTG TTG ATA CTA TGC ACA TCA CAG ATC CTC          7358
Gln Leu Gly Gln Ile Met Leu Leu Ile Leu Cys Thr Ser Gln Ile Leu
                2415                2420                2425

TTG ATG CGG ACT ACA TGG GCC TTG TGT GAA TCC ATC ACA CTG GCC ACT          7406
Leu Met Arg Thr Thr Trp Ala Leu Cys Glu Ser Ile Thr Leu Ala Thr
                2430                2435                2440

GGA CCT CTG ACC ACG CTT TGG GAG GGA TCT CCA GGA AAA TTT TGG AAC          7454
Gly Pro Leu Thr Thr Leu Trp Glu Gly Ser Pro Gly Lys Phe Trp Asn
                2445                2450                2455

ACC ACG ATA GCG GTT TCC ATG GCA AAC ATT TTC AGG GGA AGT TAT CTA          7502
Thr Thr Ile Ala Val Ser Met Ala Asn Ile Phe Arg Gly Ser Tyr Leu
        2460                2465                2470

GCA GGA GCA GGC CTG GCC TTC TCA TTA ATG AAA TCT CTA GGA GGA GGT          7550
Ala Gly Ala Gly Leu Ala Phe Ser Leu Met Lys Ser Leu Gly Gly Gly
2475                2480                2485                2490

AGG AGA GGT ACG GGA GCC AAG GGG AAA CAC TGG GAG AGA AAT GGA AAA          7598
Arg Arg Gly Thr Gly Ala Lys Gly Lys His Trp Glu Arg Asn Gly Lys
                2495                2500                2505

GAC AGA CTG AAC CAA CTG AGC AAG TCA GAA TTC AAC ACT TAC AAA AGG          7646
Asp Arg Leu Asn Gln Leu Ser Lys Ser Glu Phe Asn Thr Tyr Lys Arg
                2510                2515                2520

AGT GGG ATT ATG GAA GTG GAC AGA TCC GAA GCC AAA GAG GGA CTG AAA          7694
Ser Gly Ile Met Glu Val Asp Arg Ser Glu Ala Lys Glu Gly Leu Lys
        2525                2530                2535

AGA GGA GAA ACA ACC AAA CAT GCA GTG TCG AGA GGA ACC GCC AAA TTG          7742
Arg Gly Glu Thr Thr Lys His Ala Val Ser Arg Gly Thr Ala Lys Leu
        2540                2545                2550

AGG TGG TTC GTG GAG AGG AAC CTT GTG AAA CCA GAA GGG AAA GTC ATA          7790
Arg Trp Phe Val Glu Arg Asn Leu Val Lys Pro Glu Gly Lys Val Ile
2555                2560                2565                2570

GAC CTC GGT TGT GGA AGA GGT GGC TGG TCA TAC TAT TGC GCT GGG CTG          7838
Asp Leu Gly Cys Gly Arg Gly Gly Trp Ser Tyr Tyr Cys Ala Gly Leu
                2575                2580                2585

AAG AAA GTC ACA GAA GTG AAG GGA TAC ACA AAA GGA GGA CCT GGA CAT          7886
Lys Lys Val Thr Glu Val Lys Gly Tyr Thr Lys Gly Gly Pro Gly His
                2590                2595                2600
```

21

```
GAG GAA CCA ATC CCA ATG GCG ACC TAT GGA TGG AAC CTA GTA AAG CTA          7934
Glu Glu Pro Ile Pro Met Ala Thr Tyr Gly Trp Asn Leu Val Lys Leu
        2605              2610              2615

TAC TCC GGG AAA GAC GTA TTC TTT ACA CCA CCT GAG AAG TGT GAC ACC          7982
Tyr Ser Gly Lys Asp Val Phe Phe Thr Pro Pro Glu Lys Cys Asp Thr
    2620              2625              2630

CTT TTG TGT GAT ATT GGT GAG TCC TCT CCA AAC CCA ACT ATA GAA GAA          8030
Leu Leu Cys Asp Ile Gly Glu Ser Ser Pro Asn Pro Thr Ile Glu Glu
2635              2640              2645              2650

GGA AGA ACG TTA CGC GTC CTA AAG ATG GTG GAA CCA TGG CTC AGA GGG          8078
Gly Arg Thr Leu Arg Val Leu Lys Met Val Glu Pro Trp Leu Arg Gly
        2655              2660              2665

AAC CAA TTT TGC ATA AAA ATT CTA AAT CCC TAC ATG CCA AGT GTG GTG          8126
Asn Gln Phe Cys Ile Lys Ile Leu Asn Pro Tyr Met Pro Ser Val Val
        2670              2675              2680

GAA ACT CTG GAG CAA ATG CAA AGA AAA CAT GGA GGA ATG CTA GTG CGG          8174
Glu Thr Leu Glu Gln Met Gln Arg Lys His Gly Gly Met Leu Val Arg
        2685              2690              2695

AAT CCA CTT TCA AGA AAT TCT ACT CAT GAA ATG TAT TGG GTT TCA TGT          8222
Asn Pro Leu Ser Arg Asn Ser Thr His Glu Met Tyr Trp Val Ser Cys
        2700              2705              2710

GGA ACA GGA AAC ATT GTG TCA GCA GTA AAC ATG ACA TCT AGA ATG TTG          8270
Gly Thr Gly Asn Ile Val Ser Ala Val Asn Met Thr Ser Arg Met Leu
2715              2720              2725              2730

CTA AAT CGA TTC ACA ATG GCT CAC AGG AAA CCA ACA TAT GAA AGA GAC          8318
Leu Asn Arg Phe Thr Met Ala His Arg Lys Pro Thr Tyr Glu Arg Asp
        2735              2740              2745

GTG GAC TTA GGC GCT GGA ACA AGA CAT GTG GCA GTG GAA CCA GAG GTA          8366
Val Asp Leu Gly Ala Gly Thr Arg His Val Ala Val Glu Pro Glu Val
        2750              2755              2760

GCC AAC CTA GAT ATC ATT GGC CAG AGG ATA GAG AAC ATA AAA CAT GAA          8414
Ala Asn Leu Asp Ile Ile Gly Gln Arg Ile Glu Asn Ile Lys His Glu
        2765              2770              2775

CAT AAG TCA ACA TGG CAT TAT GAT GAG GAC AAT CCA TAT AAA ACA TGG          8462
His Lys Ser Thr Trp His Tyr Asp Glu Asp Asn Pro Tyr Lys Thr Trp
        2780              2785              2790

GCC TAT CAT GGA TCA TAT GAG GTC AAG CCA TCA GGA TCA GCC TCA TCC          8510
Ala Tyr His Gly Ser Tyr Glu Val Lys Pro Ser Gly Ser Ala Ser Ser
2795              2800              2805              2810

ATG GTC AAT GGC GTG GTG AAA CTG CTC ACC AAA CCA TGG GAT GCC ATC          8558
Met Val Asn Gly Val Val Lys Leu Leu Thr Lys Pro Trp Asp Ala Ile
        2815              2820              2825

CCC ATG GTC ACA CAA ATA GCC ATG ACT GAC ACC ACA CCC TTT GGA CAA          8606
Pro Met Val Thr Gln Ile Ala Met Thr Asp Thr Thr Pro Phe Gly Gln
        2830              2835              2840

CAG AGG GTG TTT AAA GAG AAA GTT GAC ACG CGC ACA CCA AAA GCA AAA          8654
Gln Arg Val Phe Lys Glu Lys Val Asp Thr Arg Thr Pro Lys Ala Lys
        2845              2850              2855
```

22

```
CGA GGC ACA GCA CAA ATC ATG GAG GTG ACA GCC AGG TGG TTA TGG GGT        8702
Arg Gly Thr Ala Gln Ile Met Glu Val Thr Ala Arg Trp Leu Trp Gly
    2860                2865                2870

TTT CTC TCT AGA AAC AAA AAA CCA AGA ATT TGT ACA AGA GAG GAG TTC        8750
Phe Leu Ser Arg Asn Lys Lys Pro Arg Ile Cys Thr Arg Glu Glu Phe
2875                2880                2885                2890

ACA AGA AAA GTT AGG TCA AAC GCA GCC ATT GGA GCA GTG TTC GTT GAT        8798
Thr Arg Lys Val Arg Ser Asn Ala Ala Ile Gly Ala Val Phe Val Asp
            2895                2900                2905

GAA AAT CAA TGG AAC TCA GCA AAA GAA GCA GTG GAA GAT GAG CGG TTC        8846
Glu Asn Gln Trp Asn Ser Ala Lys Glu Ala Val Glu Asp Glu Arg Phe
            2910                2915                2920

TGG GAC CTT GTG CAC AGA GAG AGG GAG CTT CAC AAA CAG GGA AAA TGT        8894
Trp Asp Leu Val His Arg Glu Arg Glu Leu His Lys Gln Gly Lys Cys
            2925                2930                2935

GCC ACG TGT GTT TAC AAC ATG ATG GGG AAG AGA GAG AAA AAA CTA GGA        8942
Ala Thr Cys Val Tyr Asn Met Met Gly Lys Arg Glu Lys Lys Leu Gly
    2940                2945                2950

GAG TTC GGA AAG GCA AAA GGA AGT CGT GCA ATA TGG TAC ATG TGG TTG        8990
Glu Phe Gly Lys Ala Lys Gly Ser Arg Ala Ile Trp Tyr Met Trp Leu
2955                2960                2965                2970

GGA GCA CGC TTT CTA GAG TTC GAA GCT CTT GGT TTC ATG AAC GAA GAT        9038
Gly Ala Arg Phe Leu Glu Phe Glu Ala Leu Gly Phe Met Asn Glu Asp
            2975                2980                2985

CAC TGG TTC AGT AGA GAG AAT TCA CTC AGT GGA GTG GAA GGA GAA GGA        9086
His Trp Phe Ser Arg Glu Asn Ser Leu Ser Gly Val Glu Gly Glu Gly
            2990                2995                3000

CTC CAC AAA CTC GGA TAT ATA CTC AGA GAC ATA TCA AAG ATT CCA GGG        9134
Leu His Lys Leu Gly Tyr Ile Leu Arg Asp Ile Ser Lys Ile Pro Gly
            3005                3010                3015

GGA AAT ATG TAT GCA GAT GAC ACA GCC GGA TGG GAT ACA AGG ATA ACA        9182
Gly Asn Met Tyr Ala Asp Asp Thr Ala Gly Trp Asp Thr Arg Ile Thr
            3020                3025                3030

GAG GAT GAT CTT CAG AAT GAG GCC AAA ATT ACT GAC ATC ATG GAG CCC        9230
Glu Asp Asp Leu Gln Asn Glu Ala Lys Ile Thr Asp Ile Met Glu Pro
3035                3040                3045                3050

GAA CAT GCC CTA CTG GCT ACG TCA ATC TTC AAG CTG ACC TAC CAA AAT        9278
Glu His Ala Leu Leu Ala Thr Ser Ile Phe Lys Leu Thr Tyr Gln Asn
            3055                3060                3065

AAG GTG GTA AGG GTA CAG AGA CCA GCG AAA AAT GGA ACC GTG ATG GAT        9326
Lys Val Val Arg Val Gln Arg Pro Ala Lys Asn Gly Thr Val Met Asp
            3070                3075                3080

GTC ATA TCC AGA CGT GAC CAG AGA GGA AGT GGC CAG GTC GGA ACT TAT        9374
Val Ile Ser Arg Arg Asp Gln Arg Gly Ser Gly Gln Val Gly Thr Tyr
            3085                3090                3095

GGC TTA AAC ACT TTC ACT AAC ATG GAA GCC CAG CTA ATA AGA CAA ATG        9422
Gly Leu Asn Thr Phe Thr Asn Met Glu Ala Gln Leu Ile Arg Gln Met
            3100                3105                3110
```

```
GAG TCT GAG GGA ATC TTT TCA CCC AGC GAA TTG GAG ACC CCA AAT TTA        9470
Glu Ser Glu Gly Ile Phe Ser Pro Ser Glu Leu Glu Thr Pro Asn Leu
3115             3120             3125             3130

GCC GAG AGA GTT CTC GAC TGG CTG GAA AAA TAT GGC GTC GAA AGG CTG        9518
Ala Glu Arg Val Leu Asp Trp Leu Glu Lys Tyr Gly Val Glu Arg Leu
            3135             3140             3145

AAA AGA ATG GCA ATC AGC GGA GAT GAC TGC GTG GTG AAA CCA ATT GAT        9566
Lys Arg Met Ala Ile Ser Gly Asp Asp Cys Val Val Lys Pro Ile Asp
            3150             3155             3160

GAC AGG TTC GCA ACA GCC TTA ACA GCT CTG AAT GAT ATG GGA AAA GTA        9614
Asp Arg Phe Ala Thr Ala Leu Thr Ala Leu Asn Asp Met Gly Lys Val
            3165             3170             3175

AGA AAA GAT ATA CCA CAA TGG GAA CCC TCA AAA GGA TGG AAT GAT TGG        9662
Arg Lys Asp Ile Pro Gln Trp Glu Pro Ser Lys Gly Trp Asn Asp Trp
    3180             3185             3190

CAA CAG GTG CCT TTT TGT TCA CAC CAT TTC CAC CAG CTG ATT ATG AAG        9710
Gln Gln Val Pro Phe Cys Ser His His Phe His Gln Leu Ile Met Lys
3195             3200             3205             3210

GAT GGG AGG GAA ATA GTG GTG CCA TGC CGC AAC CAA GAT GAA CTT GTG        9758
Asp Gly Arg Glu Ile Val Val Pro Cys Arg Asn Gln Asp Glu Leu Val
            3215             3220             3225

GGT AGG GCT AGA GTA TCA CAA GGT GCT GGA TGG AGC CTG AGA GAA ACT        9806
Gly Arg Ala Arg Val Ser Gln Gly Ala Gly Trp Ser Leu Arg Glu Thr
            3230             3235             3240

GCA TGC CTA GGC AAG TCA TAT GCA CAA ATG TGG CAG CTG ATG TAC TTC        9854
Ala Cys Leu Gly Lys Ser Tyr Ala Gln Met Trp Gln Leu Met Tyr Phe
            3245             3250             3255

CAC AGG AGA GAC CTG AGA CTA GCT GCT AAT GCT ATC TGT TCA GCC GTT        9902
His Arg Arg Asp Leu Arg Leu Ala Ala Asn Ala Ile Cys Ser Ala Val
            3260             3265             3270

CCA GTT GAT TGG GTC CCA ACC AGC CGC ACC ACT TGG TCG ATC CAT GCC        9950
Pro Val Asp Trp Val Pro Thr Ser Arg Thr Thr Trp Ser Ile His Ala
3275             3280             3285             3290

CAT CAC CAA TGG ATG ACA ACA GAA GAC ATG TTG TCA GTG TGG AAT AGG        9998
His His Gln Trp Met Thr Thr Glu Asp Met Leu Ser Val Trp Asn Arg
            3295             3300             3305

GTT TGG ATA GAG GAA AAC CCA TGG ATG GAG GAC AAA ACC CAT GTA TCC        10046
Val Trp Ile Glu Glu Asn Pro Trp Met Glu Asp Lys Thr His Val Ser
            3310             3315             3320

AGT TGG GAA GAT GTT CCA TAT TTA GGA AAA AGG GAA GAT CAG TGG TGT        10094
Ser Trp Glu Asp Val Pro Tyr Leu Gly Lys Arg Glu Asp Gln Trp Cys
            3325             3330             3335

GGA TCC CTG ATA GGC TTA ACA GCA AGG GCT ACC TGG GCC ACC AAC ATA        10142
Gly Ser Leu Ile Gly Leu Thr Ala Arg Ala Thr Trp Ala Thr Asn Ile
            3340             3345             3350

CAA GTG GCC ATA AAC CAA GTG AGA AGA CTA ATC GGG AAT GAG AAT TAT        10190
Gln Val Ala Ile Asn Gln Val Arg Arg Leu Ile Gly Asn Glu Asn Tyr
3355             3360             3365             3370
```

```
CTA GAT TAC ATG ACA TCA ATG AAG AGA TTC AAG AAC GAG AGT GAT CCG      10238
Leu Asp Tyr Met Thr Ser Met Lys Arg Phe Lys Asn Glu Ser Asp Pro
            3375                3380                3385

AAG GGG CAC TCT GGT GAG TCA ACA CAC TTA TGAAAATAAA GGAAAATAAG        10288
Lys Gly His Ser Gly Glu Ser Thr His Leu
            3390                3395

AAATCAAACA AGGCAAGAAG TCAGGCCGGA TTAAGCCATA GTACGGTAAG AGCTATGCTG    10348

CCTGTGAGCC CCGTCCAAGG ACGTAAAATG AAGTCAGGCC GAAAGCCACG GTTTGAGCAA    10408

ACCGTGCTGC CTGTAGCTTC ATCGTGGGGA TGTAAAAACC TGGGAGGCTG CAACCCATGG    10468

AAGCTGTACG CATGGGGTAG CAGACTAGTG GTTAGAGGAG ACCCCTCCCA AAACATAACG    10528

CAGCAGCGGG GCCCAACACC AGGGGAAGCT GTATCCTGGT GGTAAGGACT AGAGGTTAGA    10588

GGAGACCCCC GGCATAACAA TAAACAGCAT ATTGACGCTG GGAGAGACCA GAGATCCTGC    10648

TGTCTCTACA GCATCATTCC AGGCACAGAA CGCCAGAAAA TGGAATGGTG CTGTTGAATC    10708

AACAGGTTCT                                                          10718
```

(2) INFORMATION FOR SEQ ID NO:2:

    (i) SEQUENCE CHARACTERISTICS:

        (A) LENGTH: 3396 amino acids
        (B) TYPE: amino acid
        (D) TOPOLOGY: linear

    (ii) MOLECULE TYPE: protein

    (xi) SEQUENCE DESCRIPTION: SEQ ID NO:2:

```
Met Asn Asn Gln Arg Lys Lys Thr Ala Arg Pro Ser Phe Asn Met Leu
 1               5                  10                     15

Lys Arg Ala Arg Asn Arg Val Ser Thr Gly Ser Gln Leu Ala Lys Arg
            20                  25                  30

Phe Ser Lys Gly Leu Leu Ser Gly Gln Gly Pro Met Lys Leu Val Met
        35                  40                  45

Ala Phe Ile Ala Phe Leu Arg Phe Leu Ala Ile Pro Pro Thr Ala Gly
        50                  55                  60

Ile Leu Ala Arg Trp Gly Ser Phe Lys Lys Asn Gly Ala Ile Lys Val
 65                  70                  75                     80

Leu Arg Gly Phe Lys Lys Glu Ile Ser Asn Met Leu Asn Ile Met Asn
                85                  90                  95

Arg Arg Lys Arg Ser Val Thr Met Leu Leu Met Leu Leu Pro Thr Ala
            100                 105                 110

Leu Ala Phe His Leu Thr Thr Arg Gly Gly Glu Pro His Met Ile Val
            115                 120                 125

Ser Lys Gln Glu Arg Glu Lys Ser Leu Leu Phe Lys Thr Ser Val Gly
            130                 135                 140
```

```
Val Asn Met Cys Thr Leu Ile Ala Met Asp Leu Gly Glu Leu Cys Glu
145             150             155                     160

Asp Thr Met Thr Tyr Lys Cys Pro Arg Ile Thr Glu Ala Glu Pro Asp
                165             170                     175

Asp Val Asp Cys Trp Cys Asn Ala Thr Asp Thr Trp Val Thr Tyr Gly
            180             185                 190

Thr Cys Ser Gln Thr Gly Glu His Arg Arg Asp Lys Arg Ser Val Ala
        195             200                 205

Leu Ala Pro His Val Gly Leu Gly Leu Glu Thr Arg Thr Glu Thr Trp
    210             215                 220

Met Ser Ser Glu Gly Ala Trp Lys Gln Ile Gln Arg Val Glu Thr Trp
225             230                 235                     240

Ala Leu Arg His Pro Gly Phe Thr Val Ile Ala Leu Phe Leu Ala His
            245             250                     255

Ala Ile Gly Thr Ser Ile Thr Gln Lys Gly Ile Ile Phe Ile Leu Leu
            260             265                 270

Met Leu Val Thr Pro Ser Met Ala Met Arg Cys Val Gly Ile Gly Ser
        275             280                 285

Arg Asp Phe Val Glu Gly Leu Ser Gly Ala Thr Trp Val Asp Val Val
    290             295                 300

Leu Glu His Gly Ser Cys Val Thr Thr Met Ala Lys Asp Lys Pro Thr
305             310             315                     320

Leu Asp Ile Glu Leu Leu Lys Thr Glu Val Thr Asn Pro Ala Val Leu
            325             330                     335

Arg Lys Leu Cys Ile Glu Ala Lys Ile Ser Asn Thr Thr Thr Asp Ser
        340             345                 350

Arg Cys Pro Thr Gln Gly Glu Ala Thr Leu Val Glu Glu Gln Asp Ala
        355             360                 365

Asn Phe Val Cys Arg Arg Thr Phe Val Asp Arg Gly Trp Gly Asn Gly
    370             375                 380

Cys Gly Leu Phe Gly Lys Gly Ser Leu Leu Thr Cys Ala Lys Phe Lys
385             390             395                     400

Cys Val Thr Lys Leu Glu Gly Lys Ile Val Gln Tyr Glu Asn Leu Lys
            405             410                     415

Tyr Ser Val Ile Val Thr Val His Thr Gly Asp Gln His Gln Val Gly
            420             425                 430

Asn Glu Thr Thr Glu His Gly Thr Ile Ala Thr Ile Thr Pro Gln Ala
        435             440                 445

Pro Thr Ser Glu Ile Gln Leu Thr Asp Tyr Gly Ala Leu Thr Leu Asp
    450             455                 460

Cys Ser Pro Arg Thr Gly Leu Asp Phe Asn Glu Met Val Leu Leu Thr
465             470                 475                     480
```

27

```
Met Lys Glu Lys Ser Trp Leu Val His Lys Gln Trp Phe Leu Asp Leu
              485             490             495

Pro Leu Pro Trp Thr Ser Gly Ala Ser Thr Ser Gln Glu Thr Trp Asn
              500             505             510

Arg Gln Asp Leu Leu Val Thr Phe Lys Thr Ala His Ala Lys Lys Gln
          515             520             525

Glu Val Val Val Leu Gly Ser Gln Glu Gly Ala Met His Thr Ala Leu
          530             535             540

Thr Gly Ala Thr Glu Ile Gln Thr Ser Gly Thr Thr Thr Ile Phe Ala
545             550             555             560

Gly His Leu Lys Cys Arg Leu Lys Met Asp Lys Leu Thr Leu Lys Gly
              565             570             575

Met Ser Tyr Val Met Cys Thr Gly Ser Phe Lys Leu Glu Lys Glu Val
              580             585             590

Ala Glu Thr Gln His Gly Thr Val Leu Val Gln Val Lys Tyr Glu Gly
          595             600             605

Thr Asp Ala Pro Cys Lys Ile Pro Phe Ser Thr Gln Asp Glu Lys Gly
          610             615             620

Val Thr Gln Asn Arg Leu Ile Thr Ala Asn Pro Ile Val Thr Asp Lys
625             630             635             640

Glu Lys Pro Val Asn Ile Glu Thr Glu Pro Pro Phe Gly Glu Ser Tyr
              645             650             655

Ile Val Val Gly Ala Gly Glu Lys Ala Leu Lys Gln Cys Trp Phe Lys
              660             665             670

Lys Gly Ser Ser Ile Gly Lys Met Phe Glu Ala Thr Ala Arg Gly Ala
              675             680             685

Arg Arg Met Ala Ile Leu Gly Asp Thr Ala Trp Asp Phe Gly Ser Ile
          690             695             700

Gly Gly Val Phe Thr Ser Val Gly Lys Leu Val His Gln Val Phe Gly
705             710             715             720

Thr Ala Tyr Gly Val Leu Phe Ser Gly Val Ser Trp Thr Met Lys Ile
              725             730             735

Gly Ile Gly Ile Leu Leu Thr Trp Leu Gly Leu Asn Ser Arg Ser Thr
              740             745             750

Ser Leu Ser Met Thr Cys Ile Ala Val Gly Met Val Thr Leu Tyr Leu
          755             760             765

Gly Val Met Val Gln Ala Asp Ser Gly Cys Val Ile Asn Trp Lys Gly
          770             775             780

Arg Glu Leu Lys Cys Gly Ser Gly Ile Phe Val Thr Asn Glu Val His
785             790             795             800

Thr Trp Thr Glu Gln Tyr Lys Phe Gln Ala Asp Ser Pro Lys Arg Leu
              805             810             815
```

```
Ser Ala Ala Ile Gly Lys Ala Trp Glu Glu Gly Val Cys Gly Ile Arg
        820                 825                 830

Ser Ala Thr Arg Leu Glu Asn Ile Met Trp Lys Gln Ile Ser Asn Glu
        835                 840                 845

Leu Asn His Ile Leu Leu Glu Asn Asp Met Lys Phe Thr Val Val Val
        850                 855                 860

Gly Asp Val Val Gly Ile Leu Ala Gln Gly Lys Lys Met Ile Arg Pro
865                 870                 875                 880

Gln Pro Met Glu His Lys Tyr Ser Trp Lys Ser Trp Gly Lys Ala Lys
            885                 890                 895

Ile Ile Gly Ala Asp Ile Gln Asn Thr Thr Phe Ile Ile Asp Gly Pro
        900                 905                 910

Asp Thr Pro Glu Cys Pro Asp Asp Gln Arg Ala Trp Asn Ile Trp Glu
        915                 920                 925

Val Glu Asp Tyr Gly Phe Gly Ile Phe Thr Thr Asn Ile Trp Leu Lys
        930                 935                 940

Leu Arg Asp Ser Tyr Thr Gln Met Cys Asp His Arg Leu Met Ser Ala
945                 950                 955                 960

Ala Ile Lys Asp Ser Lys Ala Val His Ala Asp Met Gly Tyr Trp Ile
                965                 970                 975

Glu Ser Glu Lys Asn Glu Thr Trp Lys Leu Ala Arg Ala Ser Phe Ile
            980                 985                 990

Glu Val Lys Thr Cys Val Trp Pro Lys Ser His Thr Leu Trp Ser Asn
        995                 1000                1005

Gly Val Leu Glu Ser Glu Met Ile Ile Pro Lys Ile Tyr Gly Gly Pro
    1010                1015                1020

Ile Ser Gln His Asn Tyr Arg Pro Gly Tyr Phe Thr Gln Thr Ala Gly
1025                1030                1035                1040

Pro Trp His Leu Gly Lys Leu Glu Leu Asp Phe Asp Leu Cys Glu Gly
            1045                1050                1055

Thr Thr Val Val Val Asp Glu His Cys Gly Asn Arg Gly Pro Ser Leu
            1060                1065                1070

Arg Thr Thr Thr Val Thr Gly Lys Ile Ile His Glu Trp Cys Cys Arg
        1075                1080                1085

Ser Cys Thr Leu Pro Pro Leu Arg Phe Lys Gly Glu Asp Gly Cys Trp
    1090                1095                1100

Tyr Gly Met Glu Ile Arg Pro Val Lys Glu Lys Glu Glu Asn Leu Val
1105                1110                1115                1120

Lys Ser Met Val Ser Ala Gly Ser Gly Glu Val Asp Ser Phe Ser Leu
            1125                1130                1135

Gly Leu Leu Cys Ile Ser Ile Met Ile Glu Glu Val Met Arg Ser Arg
            1140                1145                1150
```

Trp Ser Arg Lys Met Leu Met Thr Gly Thr Leu Ala Val Phe Leu Leu
1155                1160              1165

Leu Ile Met Gly Gln Leu Thr Trp Asn Asp Leu Ile Arg Leu Cys Ile
1170                1175              1180

Met Val Gly Ala Asn Ala Ser Asp Arg Met Gly Met Gly Thr Thr Tyr
1185                1190              1195              1200

Leu Ala Leu Met Ala Thr Phe Lys Met Arg Pro Met Phe Ala Val Gly
1205              1210              1215

Leu Leu Phe Arg Arg Leu Thr Ser Arg Glu Val Leu Leu Leu Thr Ile
1220              1225              1230

Gly Leu Ser Leu Val Ala Ser Val Glu Leu Pro Asn Ser Leu Glu Glu
1235              1240              1245

Leu Gly Asp Gly Leu Ala Met Gly Ile Met Ile Leu Lys Leu Leu Thr
1250              1255              1260

Asp Phe Gln Ser His Gln Leu Trp Ala Thr Leu Leu Ser Leu Thr Phe
1265              1270              1275              1280

Val Lys Thr Thr Phe Ser Leu His Tyr Ala Trp Lys Thr Met Ala Met
1285              1290              1295

Val Leu Ser Ile Val Ser Leu Phe Pro Leu Cys Leu Ser Thr Thr Ser
1300              1305              1310

Gln Lys Thr Thr Trp Leu Pro Val Leu Leu Gly Ser Leu Gly Cys Lys
1315              1320              1325

Pro Leu Thr Met Phe Leu Ile Ala Glu Asn Lys Ile Trp Gly Arg Lys
1330              1335              1340

Ser Trp Pro Leu Asn Glu Gly Ile Met Ala Val Gly Ile Val Ser Ile
1345              1350              1355              1360

Leu Leu Ser Ser Leu Leu Lys Asn Asp Val Pro Leu Ala Gly Pro Leu
1365              1370              1375

Ile Ala Gly Gly Met Leu Ile Ala Cys Tyr Val Ile Ser Gly Ser Ser
1380              1385              1390

Ala Asp Leu Ser Leu Glu Lys Ala Ala Glu Val Ser Trp Glu Glu Glu
1395              1400              1405

Ala Glu His Ser Gly Ala Ser His Asn Ile Leu Val Glu Val Gln Asp
1410              1415              1420

Asp Gly Thr Met Lys Ile Lys Asp Glu Glu Arg Asp Asp Thr Leu Thr
1425              1430              1435              1440

Ile Leu Leu Lys Ala Thr Leu Leu Ala Val Ser Gly Val Tyr Pro Leu
1445              1450              1455

Ser Ile Pro Ala Thr Leu Phe Val Trp Tyr Phe Trp Gln Lys Lys Lys
1460              1465              1470

Gln Arg Ser Gly Val Leu Trp Asp Thr Pro Ser Pro Pro Glu Val Glu
1475              1480              1485

```
Arg Ala Val Leu Asp Asp Gly Ile Tyr Arg Ile Met Gln Arg Gly Leu
    1490              1495              1500

Leu Gly Arg Ser Gln Val Gly Val Gly Val Phe Gln Asp Gly Val Phe
1505              1510              1515              1520

His Thr Met Trp His Val Thr Arg Gly Ala Val Leu Met Tyr Gln Gly
              1525              1530              1535

Lys Arg Leu Glu Pro Ser Trp Ala Ser Val Lys Lys Asp Leu Ile Ser
        1540              1545              1550

Tyr Gly Gly Gly Trp Arg Phe Gln Gly Ser Trp Asn Thr Gly Glu Glu
        1555              1560              1565

Val Gln Val Ile Ala Val Glu Pro Gly Lys Asn Pro Lys Asn Val Gln
    1570              1575              1580

Thr Ala Pro Gly Thr Phe Lys Thr Pro Glu Gly Glu Val Gly Ala Ile
1585              1590              1595              1600

Ala Leu Asp Phe Lys Pro Gly Thr Ser Gly Ser Pro Ile Val Asn Arg
              1605              1610              1615

Glu Gly Lys Ile Val Gly Leu Tyr Gly Asn Gly Val Val Thr Thr Ser
        1620              1625              1630


Gly Thr Tyr Val Ser Ala Ile Ala Gln Ala Lys Ala Ser Gln Glu Gly
        1635              1640              1645

Pro Leu Pro Glu Ile Glu Asp Glu Val Phe Arg Lys Arg Asn Leu Thr
    1650              1655              1660

Ile Met Asp Leu His Pro Gly Ser Gly Lys Thr Arg Arg Tyr Leu Pro
1665              1670              1675              1680

Ala Ile Val Arg Glu Ala Ile Arg Arg Asn Val Arg Thr Leu Ile Leu
              1685              1690              1695

Ala Pro Thr Arg Val Val Ala Ser Glu Met Ala Glu Ala Leu Lys Gly
        1700              1705              1710

Met Pro Ile Arg Tyr Gln Thr Thr Ala Val Lys Ser Glu His Thr Gly
        1715              1720              1725

Lys Glu Ile Val Asp Leu Met Cys His Ala Thr Phe Thr Met Arg Leu
    1730              1735              1740

Leu Ser Pro Val Arg Val Pro Asn Tyr Asn Met Ile Ile Met Asp Glu
1745              1750              1755              1760

Ala His Phe Thr Asp Pro Ala Ser Ile Ala Arg Arg Gly Tyr Ile Ser
              1765              1770              1775

Thr Arg Val Gly Met Gly Glu Ala Ala Ala Ile Phe Met Thr Ala Thr
        1780              1785              1790

Pro Pro Gly Ser Val Glu Ala Phe Pro Gln Ser Asn Ala Val Ile Gln
        1795              1800              1805

Asp Glu Glu Arg Asp Ile Pro Glu Arg Ser Trp Asn Ser Gly Tyr Glu
    1810              1815              1820
```

```
Trp Ile Thr Asp Phe Pro Gly Lys Thr Val Trp Phe Val Pro Ser Ile
1825                1830                1835                1840

Lys Ser Gly Asn Asp Ile Ala Asn Cys Leu Arg Lys Asn Gly Lys Arg
                1845                1850                1855

Val Ile Gln Leu Ser Arg Lys Thr Phe Asp Thr Glu Tyr Gln Lys Thr
            1860                1865                1870

Lys Asn Asn Asp Trp Asp Tyr Val Val Thr Thr Asp Ile Ser Glu Met
            1875                1880                1885

Gly Ala Asn Phe Arg Ala Asp Arg Val Ile Asp Pro Arg Arg Cys Leu
        1890                1895                1900

Lys Pro Val Ile Leu Lys Asp Gly Pro Glu Arg Val Ile Leu Ala Gly
1905                1910                1915                1920

Pro Met Pro Val Thr Val Ala Ser Ala Ala Gln Arg Arg Gly Arg Ile
                1925                1930                1935

Gly Arg Asn Gln Asn Lys Glu Gly Asp Gln Tyr Val Tyr Met Gly Gln
            1940                1945                1950

Pro Leu Asn Asn Asp Glu Asp His Ala His Trp Thr Glu Ala Lys Met
            1955                1960                1965

Leu Leu Asp Asn Ile Asn Thr Pro Glu Gly Ile Ile Pro Ala Leu Phe
    1970                1975                1980

Glu Pro Glu Arg Glu Lys Ser Ala Ala Ile Asp Gly Glu Tyr Arg Leu
1985                1990                1995                2000

Arg Gly Glu Ala Arg Lys Thr Phe Val Glu Leu Met Arg Arg Gly Asp
                2005                2010                2015

Leu Pro Val Trp Leu Ser Tyr Lys Val Ala Ser Glu Gly Phe Gln Tyr
            2020                2025                2030

Ser Asp Arg Arg Trp Cys Phe Asp Gly Glu Arg Asn Asn Gln Val Leu
        2035                2040                2045

Glu Glu Asn Met Asp Val Glu Met Trp Thr Lys Glu Gly Glu Arg Lys
    2050                2055                2060

Lys Leu Arg Pro Arg Trp Leu Asp Ala Arg Thr Tyr Ser Asp Pro Leu
2065                2070                2075                2080

Ala Leu Arg Glu Phe Lys Glu Phe Ala Ala Gly Arg Arg Ser Val Ser
            2085                2090                2095

Gly Asp Leu Ile Leu Glu Ile Gly Lys Leu Pro Gln His Leu Thr Gln
        2100                2105                2110

Arg Ala Gln Asn Ala Leu Asp Asn Leu Val Met Leu His Asn Ser Glu
        2115                2120                2125

Gln Gly Gly Arg Ala Tyr Arg His Ala Met Glu Glu Leu Pro Asp Thr
    2130                2135                2140

Ile Glu Thr Leu Met Leu Leu Ala Leu Ile Ala Val Leu Thr Gly Gly
2145                2150                2155                2160
```

```
Val Thr Leu Phe Phe Leu Ser Gly Lys Gly Leu Gly Lys Thr Ser Ile
              2165              2170              2175

Gly Leu Leu Cys Val Met Ala Ser Ser Val Leu Leu Trp Met Ala Ser
              2180              2185              2190

Val Glu Pro His Trp Ile Ala Ala Ser Ile Ile Leu Glu Phe Phe Leu
         2195              2200              2205

Met Val Leu Leu Ile Pro Glu Pro Asp Arg Gln Arg Thr Pro Gln Asp
         2210              2215              2220

Asn Gln Leu Ala Tyr Val Val Ile Gly Leu Leu Phe Met Ile Leu Thr
2225              2230              2235              2240

Val Ala Ala Asn Glu Met Gly Leu Leu Glu Thr Thr Lys Lys Asp Leu
              2245              2250              2255

Gly Ile Gly His Val Ala Ala Glu Asn His His His Ala Thr Met Leu
              2260              2265              2270

Asp Val Asp Leu Arg Pro Ala Ser Ala Trp Thr Leu Tyr Ala Val Ala
         2275              2280              2285

Thr Thr Val Ile Thr Pro Met Met Arg His Thr Ile Glu Asn Thr Thr
         2290              2295              2300

Ala Asn Ile Ser Leu Thr Ala Ile Ala Asn Gln Ala Ala Ile Leu Met
2305              2310              2315              2320

Gly Leu Asp Lys Gly Trp Pro Ile Ser Lys Met Asp Ile Gly Val Pro
              2325              2330              2335

Leu Leu Ala Leu Gly Cys Tyr Ser Gln Val Asn Pro Leu Thr Leu Thr
              2340              2345              2350

Ala Ala Val Leu Met Leu Val Ala His Tyr Ala Ile Ile Gly Pro Gly
              2355              2360              2365

Leu Gln Ala Lys Ala Thr Arg Glu Ala Gln Lys Arg Thr Ala Ala Gly
         2370              2375              2380

Ile Met Lys Asn Pro Thr Val Asp Gly Ile Val Ala Ile Asp Leu Asp
2385              2390              2395              2400

Pro Val Val Tyr Asp Ala Lys Phe Glu Lys Gln Leu Gly Gln Ile Met
              2405              2410              2415

Leu Leu Ile Leu Cys Thr Ser Gln Ile Leu Leu Met Arg Thr Thr Trp
              2420              2425              2430

Ala Leu Cys Glu Ser Ile Thr Leu Ala Thr Gly Pro Leu Thr Thr Leu
         2435              2440              2445

Trp Glu Gly Ser Pro Gly Lys Phe Trp Asn Thr Thr Ile Ala Val Ser
         2450              2455              2460

Met Ala Asn Ile Phe Arg Gly Ser Tyr Leu Ala Gly Ala Gly Leu Ala
2465              2470              2475              2480

Phe Ser Leu Met Lys Ser Leu Gly Gly Gly Arg Arg Gly Thr Gly Ala
              2485              2490              2495
```

```
Lys Gly Lys His Trp Glu Arg Asn Gly Lys Asp Arg Leu Asn Gln Leu
        2500                    2505              2510

Ser Lys Ser Glu Phe Asn Thr Tyr Lys Arg Ser Gly Ile Met Glu Val
        2515                    2520              2525

Asp Arg Ser Glu Ala Lys Glu Gly Leu Lys Arg Gly Glu Thr Thr Lys
        2530                    2535              2540

His Ala Val Ser Arg Gly Thr Ala Lys Leu Arg Trp Phe Val Glu Arg
2545              2550              2555              2560

Asn Leu Val Lys Pro Glu Gly Lys Val Ile Asp Leu Gly Cys Gly Arg
            2565                    2570              2575

Gly Gly Trp Ser Tyr Tyr Cys Ala Gly Leu Lys Lys Val Thr Glu Val
            2580                    2585              2590

Lys Gly Tyr Thr Lys Gly Gly Pro Gly His Glu Glu Pro Ile Pro Met
        2595                    2600              2605

Ala Thr Tyr Gly Trp Asn Leu Val Lys Leu Tyr Ser Gly Lys Asp Val
2610                    2615              2620

Phe Phe Thr Pro Pro Glu Lys Cys Asp Thr Leu Leu Cys Asp Ile Gly
2625                    2630              2635              2640

Glu Ser Ser Pro Asn Pro Thr Ile Glu Glu Gly Arg Thr Leu Arg Val
            2645                    2650              2655

Leu Lys Met Val Glu Pro Trp Leu Arg Gly Asn Gln Phe Cys Ile Lys
            2660                    2665              2670

Ile Leu Asn Pro Tyr Met Pro Ser Val Val Glu Thr Leu Glu Gln Met
        2675                    2680              2685

Gln Arg Lys His Gly Gly Met Leu Val Arg Asn Pro Leu Ser Arg Asn
    2690                    2695              2700

Ser Thr His Glu Met Tyr Trp Val Ser Cys Gly Thr Gly Asn Ile Val
2705              2710              2715              2720

Ser Ala Val Asn Met Thr Ser Arg Met Leu Leu Asn Arg Phe Thr Met
            2725                    2730              2735

Ala His Arg Lys Pro Thr Tyr Glu Arg Asp Val Asp Leu Gly Ala Gly
        2740                    2745              2750

Thr Arg His Val Ala Val Glu Pro Glu Val Ala Asn Leu Asp Ile Ile
        2755                    2760              2765

Gly Gln Arg Ile Glu Asn Ile Lys His Glu His Lys Ser Thr Trp His
    2770                    2775              2780

Tyr Asp Glu Asp Asn Pro Tyr Lys Thr Trp Ala Tyr His Gly Ser Tyr
2785                    2790              2795              2800

Glu Val Lys Pro Ser Gly Ser Ala Ser Ser Met Val Asn Gly Val Val
            2805                    2810              2815

Lys Leu Leu Thr Lys Pro Trp Asp Ala Ile Pro Met Val Thr Gln Ile
        2820                    2825              2830
```

34

```
Ala Met Thr Asp Thr Thr Pro Phe Gly Gln Gln Arg Val Phe Lys Glu
    2835                2840                2845

Lys Val Asp Thr Arg Thr Pro Lys Ala Lys Arg Gly Thr Ala Gln Ile
    2850                2855                2860

Met Glu Val Thr Ala Arg Trp Leu Trp Gly Phe Leu Ser Arg Asn Lys
2865                2870                2875                2880

Lys Pro Arg Ile Cys Thr Arg Glu Glu Phe Thr Arg Lys Val Arg Ser
                2885                2890                2895

Asn Ala Ala Ile Gly Ala Val Phe Val Asp Glu Asn Gln Trp Asn Ser
            2900                2905                2910

Ala Lys Glu Ala Val Glu Asp Glu Arg Phe Trp Asp Leu Val His Arg
        2915                2920                2925

Glu Arg Glu Leu His Lys Gln Gly Lys Cys Ala Thr Cys Val Tyr Asn
    2930                2935                2940

Met Met Gly Lys Arg Glu Lys Lys Leu Gly Glu Phe Gly Lys Ala Lys
2945                2950                2955                2960

Gly Ser Arg Ala Ile Trp Tyr Met Trp Leu Gly Ala Arg Phe Leu Glu
            2965                2970                2975

Phe Glu Ala Leu Gly Phe Met Asn Glu Asp His Trp Phe Ser Arg Glu
            2980                2985                2990

Asn Ser Leu Ser Gly Val Glu Gly Glu Gly Leu His Lys Leu Gly Tyr
    2995                3000                3005

Ile Leu Arg Asp Ile Ser Lys Ile Pro Gly Gly Asn Met Tyr Ala Asp
    3010                3015                3020

Asp Thr Ala Gly Trp Asp Thr Arg Ile Thr Glu Asp Asp Leu Gln Asn
3025                3030                3035                3040

Glu Ala Lys Ile Thr Asp Ile Met Glu Pro Glu His Ala Leu Leu Ala
            3045                3050                3055

Thr Ser Ile Phe Lys Leu Thr Tyr Gln Asn Lys Val Val Arg Val Gln
            3060                3065                3070

Arg Pro Ala Lys Asn Gly Thr Val Met Asp Val Ile Ser Arg Arg Asp
    3075                3080                3085

Gln Arg Gly Ser Gly Gln Val Gly Thr Tyr Gly Leu Asn Thr Phe Thr
    3090                3095                3100

Asn Met Glu Ala Gln Leu Ile Arg Gln Met Glu Ser Glu Gly Ile Phe
3105                3110                3115                3120

Ser Pro Ser Glu Leu Glu Thr Pro Asn Leu Ala Glu Arg Val Leu Asp
            3125                3130                3135

Trp Leu Glu Lys Tyr Gly Val Glu Arg Leu Lys Arg Met Ala Ile Ser
        3140                3145                3150

Gly Asp Asp Cys Val Val Lys Pro Ile Asp Asp Arg Phe Ala Thr Ala
    3155                3160                3165
```

```
Leu Thr Ala Leu Asn Asp Met Gly Lys Val Arg Lys Asp Ile Pro Gln
    3170                3175                3180

Trp Glu Pro Ser Lys Gly Trp Asn Asp Trp Gln Gln Val Pro Phe Cys
    3185                3190                3195                3200

Ser His His Phe His Gln Leu Ile Met Lys Asp Gly Arg Glu Ile Val
                3205                3210                3215

Val Pro Cys Arg Asn Gln Asp Glu Leu Val Gly Arg Ala Arg Val Ser
                3220                3225                3230

Gln Gly Ala Gly Trp Ser Leu Arg Glu Thr Ala Cys Leu Gly Lys Ser
                3235                3240                3245

Tyr Ala Gln Met Trp Gln Leu Met Tyr Phe His Arg Arg Asp Leu Arg
                3250                3255                3260

Leu Ala Ala Asn Ala Ile Cys Ser Ala Val Pro Val Asp Trp Val Pro
3265                3270                3275                3280

Thr Ser Arg Thr Thr Trp Ser Ile His Ala His His Gln Trp Met Thr
                3285                3290                3295

Thr Glu Asp Met Leu Ser Val Trp Asn Arg Val Trp Ile Glu Glu Asn
                3300                3305                3310

Pro Trp Met Glu Asp Lys Thr His Val Ser Ser Trp Glu Asp Val Pro
                3315                3320                3325

Tyr Leu Gly Lys Arg Glu Asp Gln Trp Cys Gly Ser Leu Ile Gly Leu
                3330                3335                3340

Thr Ala Arg Ala Thr Trp Ala Thr Asn Ile Gln Val Ala Ile Asn Gln
3345                3350                3355                3360

Val Arg Arg Leu Ile Gly Asn Glu Asn Tyr Leu Asp Tyr Met Thr Ser
                3365                3370                3375

Met Lys Arg Phe Lys Asn Glu Ser Asp Pro Lys Gly His Ser Gly Glu
                3380                3385                3390

Ser Thr His Leu
    3395
```

(2) INFORMATION FOR SEQ ID NO:3:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:3:

```
CCATGAATTC CCATGCGATG CGTGGGA                                    27
```

(2) INFORMATION FOR SEQ ID NO:4:

(i) SEQUENCE CHARACTERISTICS:

(A) LENGTH: 27 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:4:

CACATCTCGA GTCCGCTTGA ACCATGA                                    27

(2) INFORMATION FOR SEQ ID NO:5:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:5:

TGGTTCCCGG GGACTCGGGA TGTGTA                                     26

(2) INFORMATION FOR SEQ ID NO:6:

(i) SEQUENCE CHARACTERISTICS:

(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:6:

ACTAAGCTTG ATCATGCAGA GACCATTGA                                  29

(2) INFORMATION FOR SEQ ID NO:7:

(i) SEQUENCE CHARACTERISTICS:

(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:7:

```
AATCAGAATT CTCTGCAGGG TCAGGGGAA                                              29
```

(2) INFORMATION FOR SEQ ID NO:8:

    (i) SEQUENCE CHARACTERISTICS:

        (A) LENGTH: 30 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:8:

```
ATAACAAAGC TTATCTTTGT TTCTTTTTCT                                             30
```

(2) INFORMATION FOR SEQ ID NO:9:

    (i) SEQUENCE CHARACTERISTICS:

        (A) LENGTH: 31 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:9:

```
GAAAGGATCC TCTGGAGTGT TATGGGACAC A                                           31
```

(2) INFORMATION FOR SEQ ID NO:10:

    (i) SEQUENCE CHARACTERISTICS:

        (A) LENGTH: 27 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:10:

```
ACCCAAGCTT CATCTTCTTC CTGCTGC                                                27
```

(2) INFORMATION FOR SEQ ID NO:11:

    (i) SEQUENCE CHARACTERISTICS:

        (A) LENGTH: 25 base pairs
        (B) TYPE: nucleic acid

    (C) STRANDEDNESS: single
    (D) TOPOLOGY: linear

(ii) MOLECULE TYPE: DNA (genomic)

(xi) SEQUENCE DESCRIPTION: SEQ ID NO:11:


**AGGAGGTCGA CGAGGTACGG GAGCC**          25


(2) INFORMATION FOR SEQ ID NO:12:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:12:


**CAATGATATC TAGGTTGGCT**          20


## Description

The present invention relates to Dengue Virus Type 1. Dengue virus infection may lead to dengue fever (DF) or its more severe dengue haemorrhagic fever (DHF) and dengue shock syndrome (DSS). DHF is an important virus disease of global significance, especially in Southeast Asia. There are four serotypes of Denque virus (DEN1, DEN2, DEN3 and DEN4) belonging to the family Flaviviradae.

The complete genomic sequence of DEN2 (Jamaica) has been published by Deubel et al; Virology 165, 234-244 (1988). The complete genomic sequence of DEN3 (H87) has been published by Osatomi and Sumiyoshi; Virology 176, 643-647 (1990). The complete genomic sequence of DEN4 has been published by Zhao et al; Virology 155, 77-88. To date, only a partial sequence of any variant of DEN1, DEN1 (Nauru Island), has been determined; Mason et al, Virology 161, 262-267 (1987).

We have now identified a previously unknown strain of DEN1 and established its complete nucleotide sequence. The new strain, DEN1-S275/90, was deposited at the European Collection of Animal Cell Cultures (ECACC) Porton Down, GB under Budapest Treaty conditions on 21 April 1992 and given accession number V92042111. DEN1-S275/90 differs significantly from DEN2, DEN3 and DEN4 in terms of sequence homology. There are also a number of significant differences between DEN1-S275/90 and DEN1 (Nauru Island).

The present invention thus provides DEN1-S275/90 (ECACC V92042111). The invention further provides DEN1-S275/90 (ECACC V92042111) for use as a diagnostic reagent. The invention also provides DEN1-S275/90 in inactivated form for use as a diagnostic reagent or a vaccine.

The invention also provides the nucleic acid sequence of Seq. ID No. 1 and DNA sequences substantially corresponding to SEQ ID No. 1, e.g. degenerate variants thereof having one or more nucleotide changes but nevertheless capable of being translated to give the same protein sequence. The invention further provides fragments of such DNA polynucleotides, in particular the fragments encoding the C, C',PreM, M, E, NS1, NS2A, NS2B, NS3, NS4A, NS4B and NS5 genes of the genome of the virus. The start and end points of these preferred fragments in the nucleic acid sequence of Seq I.D. No. 1 are shown below in Table 1. Table 1 also shows the start and end points of the proteins encoded by these genes, using the numbering of Seq. ID Nos. 1 and 2.

TABLE 1

| Start and end points of the nucleic acid (n) numbers encoding the genes of S275/90. The table also shows the start and end points of the corresponding proteins (p) within the polyprotein encoded by S275/90. | | | | |
|---|---|---|---|---|
| Gene | Start(n) | End(n) | Start(p) | End(p) |
| C | 81 | 422 | 1 | 114 |
| C' | 123 | 422 | 15 | 114 |
| PreM | 423 | 695 | 115 | 205 |
| M | 696 | 920 | 206 | 280 |
| E | 921 | 2402 | 281 | 774 |
| NS1 | 2403 | 3464 | 775 | 1128 |
| NS2A | 3465 | 4112 | 1129 | 1344 |
| NS2B | 4113 | 4499 | 1345 | 1474 |
| NS3 | 4500 | 6359 | 1475 | 2093 |
| NS4A | 6360 | 6809 | 2094 | 2242 |
| NS4B | 6810 | 7556 | 2243 | 2492 |
| NS5 | 7557 | 10268 | 2493 | 3396 |

The nucleic acid sequences of the invention may be used as probes in an assay to determine the presence or absence of DEN1-S275/90, or they may be incorporated into a vector, eg. an expression vector.

Nucleic acid fragments according to the invention may be made by known methods of chemical synthesis or cloned from the virus itself using known recombinant techniques. Fragments according to the invention may also be produced by replication of DNA or RNA, by transcription from DNA to form RNA fragments or reverse transcription from RNA fragments to form DNA fragments. Such transcription may be in a cell free system or may be effected in cells for instance by cloning. Cell free systems include an appropriate replicase, transcriptase or reverse transcriptase, suitable nucleotide precursors and a nucleic acid template or appropriate sequence, together with buffers and any necessary or desirable cofactors.

The present invention also provides a polyprotein as set forth in Seq. ID No. 1 and Seq. ID No. 2 and fragments thereof, eg. the C, C', PreM, M, E, NS1, NS2A, NS2B, NS3, NS4A, NS4B and NS5 proteins as identified above in Table 1. The invention thus provides a polypeptide having an amino acid sequence substantially corresponding to the sequence shown in SEQ ID No. 2 or a fragment thereof. Fusion proteins which incorporate these peptides are also provided.

The polyprotein and proteins according to the invention may be produced by synthetic peptide chemistry or by expressing vectors carrying DNA encoding the proteins in a suitable cell in order to produce expression of the DNA, followed by recovery of the expressed protein. Methods of expressing and recovering recombinant proteins, including fusion proteins, are well known in the art.

For example, for expression of a polypeptide of the invention, an expression vector may be constructed. An expression vector is prepared which comprises a DNA sequence encoding a polypeptide of the invention and which is capable of expressing the polypeptide when provided with a suitable host, eucaryotic or procaryotic. Appropriate transcriptional and translational control elements are provided, including a promoter for the DNA sequence, a transcriptional termination site, and translational start and stop codons. The DNA sequence is provided in the correct frame such as to enable expression of the polypeptide to occur in a host compatible with the vector. The expression vector may be selected to be suitable to express the nucleic acid sequences of the invention in, for example, a bacterial e.g. E. coli, yeast, insect or mammalian cell. A baculovirus expression system may be used. The nucleic acid may be expressed in order that a protein or peptide encoded by the fragment alone is produced or alternatively it may be expressed to provide a fusion protein in which DEN1-S275/90 or a protein thereof, e.g. E, NS1, NS2, NS3 or NS5 as identified in Table 1 above is fused to a second amino acid sequence, e.g. a C-terminal sequence derived from glutathione S-transferase or maltose binding protein or a C-terminal or N-terminal signal sequence. Such a sequence may for example cause the fusion protein to be exported from the cell. The expression vector is then provided with an appropriate host. Cells harbouring the vector are grown so as to enable expression to occur. The vector may be a plasmid or a viral vector.

Recovery and where desirable, further purification of the protein produced by an expression vector in a host cell may be by means known in the art. Such means are designed to separate the protein of the invention from the other proteins of the host cell. Suitable means include chromatographic separation of the recovered protein.

The polyprotein and peptides of the invention may be used as immunogens for a vaccine against DEN1-S275/90 and other DEN1 viruses. Suitably, the proteins and peptides of the invention will be combined with a pharmaceutically acceptable carrier or diluent in order to prepare a sterile vaccine composition. The vaccine composition may then be used in a method of immunizing a human against DEN1 infections.

Advantageously, a vaccine composition against DEN1 may comprise a mixture of two or more peptides. For example, it may comprise one non-structural (NS) peptide, eg. NS1 or NS3, together with a capsid (C), M or E peptide. A mixture of two or more NS peptides could also be used.

The proteins and peptides of the invention may also be used as antigens in an immunoassay to detect the presence or absence of DEN1, and especially DENI-S275/90. The proteins and peptides are optionally labelled with a detectable label, eg a radioisotope, biotin or a fluorophore. The immunoassay may be conducted by bringing a known quantity of labelled protein (antigen) into contact with a sample suspected of containing antibody against DEN1 and detecting the presence or absence of antibody-antigen complex containing the labelled antigen.

The invention also provides antibodies against the above-mentioned proteins and peptides of the invention. The antibodies may be monoclonal or polyclonal. Monoclonal antibodies may be produced by hybridoma techniques known in the art or by recombinant means to provide hybrid antibodies such as humanized antibodies.

The antibodies of the invention may be used in a method of treatment, eg passive immunisation, of DEN1 infections. The antibodies may also be used in a method of diagnosis, eg by immunoassay, to detect the presence or absence of DEN1 in a sample. The antibodies may be labelled as described above for the proteins and peptides of the invention. They may also be labelled with a toxin or isotope selected to kill virus-infected cells. Antibodies against NS1 are particularly favoured since NS1 is expressed on the surface of Dengue virus-infected cells.

The antibodies of the invention may also be used in a method to detect the presence or absence of DEN1 protein in a sample. The method may comprise bringing the antibody into contact with a sample suspected to contain DEN1 proteins (antigens) and detecting the amount of antibody-antigen complex formed. Immunoassays according to the invention may be, for example, competitive (eg radioimmune assays - RIA) or non-competitive (eg enzyme linked immunosorbent assays - ELISA).

The following Examples illustrate the invention. In the accompanying drawings:

Figure 1 is a diagrammatic representation of the cDNA of Dengue virus Type 1 (Singapore strain S275/90) and fragments of said DNA in expression vectors;

Figure 2 shows gel results confirming serologic responses in mice after immunisations with fusion proteins prepared as in Examples 2 - 5 with or without complete Freund's adjuvant (CFA).

Gel Lanes:    Lane 1: M, Lane 2: anti E, Lane 3: anti-E+ CFA, Lane 4: anti-NS1, Lane 5: anti-NS2, Lane 6: anti-NS2+ CFA, Lane 7: anti-NS3, Lane 8: anti-NS3+ CFA, Lane 9: anti-NS5, Lane 10: anti-NS5+ CFA, Lane 11: positive rabbit sera, Lane 12: negative rabbit sera, Lane 13: M;

Figure 3 shows gel results confirming serologic response in rabbits after immunisations with fusion proteins prepared as in Examples 2 to 5. (-), serum before immunisation; (+) serum after immunisation.

Gel Lanes:    Lane 1: (-), Lane 2: (+) anti-E, Lane 3: (-), Lane 4: (+) anti-NS1, Lane 5: (-), Lane 6: (+) anti-NS2, Lane 7: (-), Lane 8: (+) anti-NS3, Lane 9: (-), Lane 10: (+) anti-NS5, Lane 11: positive Dengue, Lane 12: patient sera;

Figure 4 shows fluorescence microscopy of C6/36 cells infected with Dengue Type 1 DI-275 and probed with antibodies against recombinant fusion proteins. A, control antiserum; B, anti-E; C, anti-NS1; D, anti-NS2; E, anti-NS3; F anti-NS5.

## EXAMPLE 1

DEN1 virus, strain S275/90, was isolated in 1990 from the serum of a DHF patient in Singapore by 3 passages in AP61 *(Aedes psuedoscutellaris)* cells followed by 3 passages in C6/36 *(Aedes albopictus)* cells, and identified by immunofluorescence using type-specific monoclonal antibodies. After a further 8 to 13 passages in C6/36 cells, the virus-infected culture fluid was partially purified by precipitation with polyethylene glycol and ultracentrifugation on a 30% sucrose cushion (6). The viral RNA was extracted from the purified virus by treatment with phenol in the presence of sodium dodecyl sulphate. Following cDNA synthesis (cDNA Synthesis System Plus, Amersham) using random primers, the assorted cDNAs were cloned into *EcoRI* sites of pUC18 vector via *EcoRI* adaptors (Promega). The *Esherichia coli* transformants containing Dengue-specific sequences were screened by colony hybridisation with $^{32}$P-labelled cDNA probes prepared by reverse transcription of strain S275/90 RNA. The cloning procedure yielded overlapping cDNA clones containing inserts ranging in size from 0.5kb to 2.7kb. The ends of these primary clones and their subclones obtained by nested deletional analysis (Erase-a-Base System, Promega) were subjected to double-strand sequencing (Sequenase Version 2.0, United States Biochemical). The sequence data generated covers about 90% of the genomic sequence of S275/90.

Potential secondary structures have been postulated for the 5' and 3' ends of flaviviruses (4, 7, 8), posing a problem in obtaining clones with intact ends. A different stragegy for sequencing the 5' and 3' noncoding regions was used to increase the chances of obtaining clones which contain these sequences as well as the terminal end sequences of the genome. cDNAs of strain S275/90 were obtained by random priming and oligo(dT) priming (after poly(A) tailing of the virus RNA]; these were amplified by polymerase chain reaction (PCR) in the presence of specific primers, 796 and 10090/B, respectively. The cDNAs of interest were then religated into pUC18 vector. The nucleotide sequences of the primers are as follows: primer 796, 5' CCG TGA ATC CTG GGT GTC 3'; primer 10090/B, 5' GGG AAT TCC AGT GGT GTG GATC 3' with a *Bam*HI site at its 5' end. The sequences of the primers were selected from that of the initial clones of strain S275/90. To obtain the sequences at the 5' noncoding region, random cDNA clones were first generated as described above, followed by ligation to *Eco*RI adaptors before insertion into the EcoRI sites of the pUC18 vector. These ligated products of assorted cDNA inserts were flanked by the reverse and forward sequencing primers of M13 in the pUC18 vector. The forward sequencing primer was thus used as one of the primers for PCR. The ligated cDNA clones were used as templates for PCR in the presence of primer 796 (which binds to the plus strand of the template at nucleotide position 808 to 825 of strain S275/90) and the commercial M13 single-strand primer (5'GTA AAA CGA CGG CCAGT 3', Pharmacia). The amplified cDNAs thus contained the polylinker from the pUC18 vector at one end and an Xbal site (at nucleotide position 728) at the other end. For the 3' noncoding region, an additional step was included before cDNA synthesis. After extraction, the purified Dengue viral RNA was tailed by poly A polymerase (Bethesda Research Laboratories) with ATP. This was followed by cDNA synthesis using oligo(dT) as primer for the first strand cDNA synthesis. The same procedures of EcoRI adaptors ligation and insertion into EcoRI sites of the pUC18 vector were repeated. The ligated products were again subjected to PCR amplification using the primer 10090/B (which binds to the minus strand of the template at nucleotide positions 10,086 to 10,099 of strain S275/90) and the commercial M13 single-strand primer.

All samples were amplified by 30 cycles of PCR with melting, annealing and polymerisation conditions of 1 minute at 94°C, 2 minutes at 55°C and 3 minutes at 72°C, respectively. The amplified DNA was purified by electroelution in agarose gel followed by appropriate restriction enzyme digestions. The PCR amplified cDNAs at the 5' noncoding region were double digested with *Xba*l and *Eco*RI, while those at the 3' noncoding region were digested with BamHI and *Eco*lR before cloning into the appropriate sites of the pUC18 vector. The clones were screened and subjected to double-strand sequencing as described above.

The sequence data obtained from the overlapping cDNA clones was ordered by homology alignment with the published sequences of the four Dengue serotypes DEN1, DEN2, DEN3 and DEN4 using the computer program of Wilbur and Lipman (9). Seq ID No. 1 shows the complete nucleotide sequence of strain S275/90, which is 10,718 nucleotides in length, and its deduced amino acid sequence. The reading frame begins with the first AUG start codon, corresponding to nucleotides 81 to 83, and contains an open reading frame of 10,188 nucleotides encoding a polyprotein of 3396 amino acids; there are 80 nucleotides in the 5' noncoding region and 450 nucleotides in the 3' noncoding region. The sequence in the 5' noncoding region preceding the first AUG codon of the open reading frame appears to be conserved for all Dengue virus types (1-4). The length of the 3' noncoding region of strain S275/90 is longer than that of DEN2 (412 nucleotides), DEN3 (433 nucleotides) and DEN4 (384 nucleotides).

The nucleotide composition of strain S275/90 is 31.9% A, 25.9% G, 21.5% T and 20.7% C. As reported for the other flaviviruses, the same purine-rich composition was observed, and there is an absence of poly(A) tract at the 3' end.

The individual protein coding segments are based on comparison with protein sequence data for all the proteins determined from the four Dengue serotypes. These cleavage sites may reveal the involvement of viral or cellular proteases involved in protein processing. The C, preM, M, E, NS1, NS2A, NS2B, NS3, NS4A, NS4B and NS5 proteins are cleaved at the sites M/MNQRKK, A/FXL, RXKR/SV, X/MRCXG, VQA/DXGCV, VXA/GXG, X/SWPLN, KXQR/XG, GRX/S, VXA/NE and R/G, respectively, where X refers to any residue. The cleavage sites of NS2A, NS3 and NS4B conform to the reported consensus sequences (4, 5), which were originally established by Rice *et al* (10).

The nucleotide sequences of the structural and nonstructural regions (5' noncoding end to NS1, about 2400 nucleotides in length) of Nauru Island strain of DEN1 (isolated in 1974) and strain S275/90 were compared. Nucleotide variation shows that transitions are about 85.0% [transitions/(transitions + transversions) x 100%] in the structural region and 92.1% in the NS1 region; 15% of these base changes are transversions in the structural region and 7.9% in the NS1 region. The overall 236 nucleotide differences have given rise to 27 amino acid substitutions. As shown in Table 2, the nucleotide homology is 93.1% and when translated, the amino acid homology is 97.6%. Although both strains were isolated from different geographic regions with an interval of 16 years, a higher homology was still observed between the two strains. It can also be seen in Table 2 that strain S275/90 shows a higher homology with DEN3 then with DEN2 and DEN4. The nucleotide divergence of each gene is less than the translated amino acid divergence. The greatest nucleotide and amino acid changes, and hence the greatest evolution, lie in the nonstructural gene NS2A in all the four Dengue serotypes. A high homology is found in NS3 and NS5, which contain conserved sequences.

Chu *et al* (11) compared three topotypes of DEN1 strains (Thailand, Philippines and Caribbean) genetically at the envelope region. They found nucleotide changes to be less than 5% but translational differences of 2% at the amino acid level. Our strain S275/90 shows nucleotide changes of 7.7% and amino acid changes of 2.6% in the envelope region. Rico-Hesse (6) compared nucleotide sequences within a chosen E/NS1 region to estimate evolutionary relationships among 40 DEN1 strains of different geographic range and time period.

TABLE 2

| HOMOLOGY (%) COMPARISON OF ALIGNED NUCLEOTIDE SEQUENCES OF THE FOUR DENGUE SEROTYPES WITH STRAIN S275/90 (AMINO ACID ALIGNMENT WITHIN BRACKETS). | | | | |
|---|---|---|---|---|
| S275/90 | DEN1 | DEN2 | DEN3 | DEN4 |
| Full length | 93.1 (97.6) | 67.1 (70.9) | 70.4 (75.5) | 65.1 (67.6) |
| 5' non-coding | 100 | 81.7 | 93.8 | 87.7 |
| C | 97.4 (98.2) | 70.5 (67.5) | 80.5 (80.7) | 68.1 (67.9) |
| PrM | 91.6 (95.6) | 71.1 (75.8) | 75.8 (78.0) | 68.0 (68.1) |
| M | 93.3 (98.7) | 64.0 (70.7) | 70.3 (78.7) | 60.7 (60.3) |
| E | 92.3 (97.4) | 65.4 (67.7) | 69.0 (76.4) | 64.8 (61.8) |
| NS1 | 92.6 (98.0) | 70.1 (73.6) | 74.5 (78.7) | 70.1 (68.8) |
| NS2A | - | 55.1 (39.0) | 57.0 (46.8) | 51.7 (37.9) |
| NS2B | - | 66.0 (60.8) | 69.4 (69.2) | 63.1 (60.8) |
| NS3 | - | 72.0 (79.3) | 74.0 (84.5) | 69.9 (75.4) |
| NS4A | - | 63.0 (61.4) | 69.6 (68.7) | 62.8 (58.7) |
| NS4B | - | 69.7 (76.7) | 74.9 (82.3) | 71.1 (75.9) |
| NS5 | - | 71.7 (78.7) | 73.8 (81.0) | 69.9 (72.9) |
| 3' non-coding | - | 83.8 | 87.4 | 79.5 |

## REFERENCES

1. WESTAWAY, E.G., BRINTON, M.A., GAIDAMOVICH, S. Ya, HORZINEK, M.C., IGARASHI, A., KAARIAINEN, L., LVOV, D.K., PORTERFIELD, J.S., RUSSEL, P.K., and TRENT, D.W., Intervirology 24, 183-192 (1985).
2. ROSEN, L., Supplement to S. Afr. J. Med. 11, 40-42 (1986).
3. HALSTEAD, S., Science 239, 476-481 (1988).
4. DEUBEL, V., KINNEY, R.M., and TRENT, D.W., Virology 165, 234-244 (1988).
5. OSATOMI, K. and SUMIYOSHI, H., Virology 176, 643-647 (1990).
6. RICO-HESSE, R., PALLANSCH, M.A., NOTTAY, B.K., AND KEW, O.M., Virology 174, 479-493 (1990)
7. BRINTON, M.A., and DISPOTO, J.H., Virology 162, 290-299 (1988).
8. IRIE, K., MOHAN, P.M., SASAGURI, Y., PUTNAK, R. and PADMANABHAN, R., Gene 75, 197-211 (1989).
9. WILBUR, W.J., and LIPMAN, D.J., Proc. Natl. Acad. Sci. USA 80, 726-730 (1983).
10. RICE, C.M., LENCHES, E.M., EDDY, S.R., SHIN, S.J., SHEETS, R.L., and STRAUSS, J.H., Science 229, 726-733 (1985).
11. CHU, M.C., O'ROURKE, E.J. and TRENT, D.W., J. Gen. Virol. 70, 1701-1712 (1989).

## EXAMPLE 2

### CONSTRUCTION OF EXPRESSION PLASMIDS

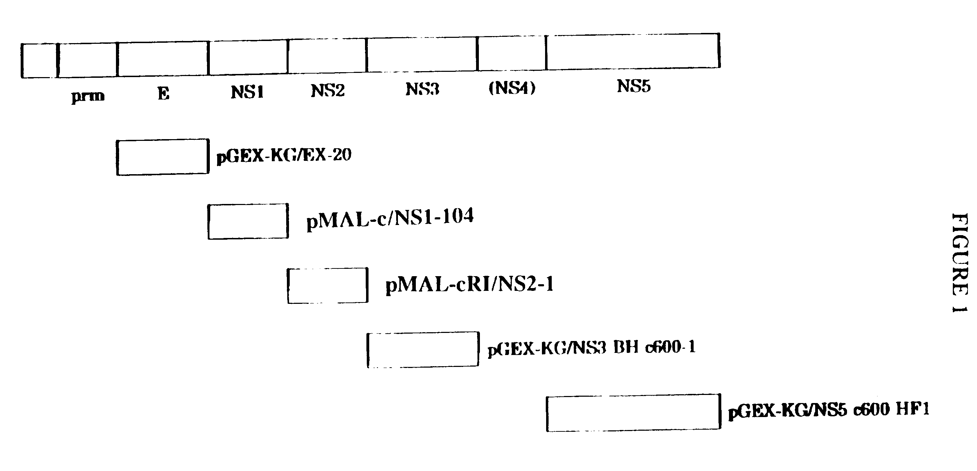

Standard recombinant DNA techniques were used for construction of the expression plasmids described below and summarised in Fig. 1 (Sambrook et al., Molecular Cloning: a laboratory manual. Cold Spring Harbor Laboratory Press, N.Y.).

For construction of plasmids, the cDNA regions for E, NS1, NS2, NS3 and NS5 of clone DI-275, a DEN1 cDNA clone derived from DEN1 virus Singapore Strain S275/90 as in Example 1, were amplified by the polymerase chain reaction (PCR) and digested with restriction enzymes. The restriction enzyme sites were built into the oligonucleotide primers used in the PCR as set out in Table 3 and Seq ID Nos. 3-12.

Fragments of E, NS3 and NS5 cDNA digested with restriction enzymes were ligated to the pGEX-KG vector (Guan and Dixon, Anal. Biochem. 192, 262-267, 1991). Fragments of NS1 and NS2 cDNA were ligated to pMAL-c and pMAL-cRI vectors (New England Biolabs), respectively (Ford et al., Prot. Exp. Pur. 2, 96-107, 1991; Maina et al., Gene 74, 365-373, 1988; di Guan et al., Gene, 67, 21-30, 1991). The construction of NS5 cDNA was done in two stages. The 5'-region, the cDNA fragment from nucleotide 7544-8365 of NS5, was made by PCR, digested with SalI and ClaI; and the 3'-region, the fragment from nucleotide 8275 (ClaI) to the 3'-end of NS5, was isolated directly from the cDNA of clone DI-275 (D-275 cDNA) by ClaI and SacI double digestion. The two parts of NS5 were ligated together, then ligated into the pGEX-KG vector. Recombinant plasmids were transformed into E. coli DH5a or c600 HF1 strains. All plasmids encoded Dengue virus proteins fused to the C-terminus of glutathione S-transferase or Maltose Binding Protein (MBP).

## EXAMPLE 3

### PURIFICATION OF E, NS3 AND NS5 PROTEINS FROM RECOMBINANT

### E. COLI

E. coli, harbouring E, NS3 and NS5 genes (separately) were grown in LB medium $A_{600}$ of 0.5 at 37°C, then induced with IPTG at 0.2mM for 2 h at 30°C. The bacteria were harvested and resuspended on ice in MTPBS buffer (0.15 M NaCl, 0.016 M $Na_2HPO_4$, 0.005 M $NaH_2O_4$) with 0.1 mg/ml lysozyme, 1% triton X-100, 0.5 μg/ml aprotinin, 0.05 μg/ml Leupeptin, 0.25 μg/ml pepstatin, 5mM DTT and 0.175 μg/ml PMSF, and kept on ice for 10 min. The cells were sonicated at maximum power for 3 x 1 min while chilled. The lysate was centrifuged at 12,000 x g. The supernatant was added to 1 ml Glutathione-Sepharose 4B beads (Pharmacia), and incubated at 4°C on a rotator for 1 h to absorb the fusion proteins. Then the beads were centrifuged and washed with PBS buffer (by centrifugation) at least 6 times, or until the wash solution read zero at $A_{280}$ in a spectrophotometer. The beads were resuspended in thrombin cleavage buffer, and the Dengue virus proteins were cleaved off the beads with thrombin at 4°C for 1 hr. The supernatant, containing Dengue virus proteins, was recovered by centrifugation, and the proteins were stored at -80°C.

EXAMPLE 4

**SOLUBILISATION AND PURIFICATION OF A FUSION PROTEIN OF NS1 FROM INCLUSION BODIES**

*E. coli* containing the NS1 fusion protein was grown as above, except the tac promoters were induced with 0.3mM IPTG for 16 h. The bacteria were harvested, 1 gram wet weight of *E. coli* was resuspended in 5 ml lysis buffer with lysozyme at 1.6 mg/ml and was sonicated for 2 x 15 sec. After centrifugation at 1000 x g the supernatant was again centrifuged (25,000 x g). The pellet was resuspended in 2 ml $H_2O$ , adding a final concentration of 0.5% Triton X-100, 10 mM EDTA, and 100 mM NaCl, then centrifuged at 20,000 x g twice. The pellet was washed with 1 ml 2 M urea twice and dissolved in 8 M urea in 0.1 M Tris-HCl pH 8.8, 0.14 M 2-mercaptoethanol. The urea concentration was reduced to 1 M by adding $H_2O$, and amylose resin (New England Biolabs) was added to adsorb the solubilised fusion protein at 22°C for 1 h. The amylose resin was washed with buffer (New England Biolabs) five times until the $A_{280}$ of the clarified supernatant was near zero. A final concentration of 50 mM maltose was then added to elute the fusion protein, which was recovered by removing the beads by centrifugation.

EXAMPLE 5

**PURIFICATION OF A SOLUBLE FUSION PROTEIN OF NS2**

After growth of *E. coli* transformed with pMAL-cRI/NS2-1, lysis and sonication as in Example 3 above, the clarified extract containing the soluble NS2 fusion protein was adsorbed onto amylose resin, followed by washing and elution of the NS2 fusion protein as in Example 4 above.

EXAMPLE 6

**IMMUNISATION OF RABBITS AND MICE**

The soluble fusion proteins of E, NS2, NS3 and NS5 purified from recombinant *E. coli*, as in Examples 3 and 5 above, and inclusion bodies containing the NS1 fusion protein which had been purified up to the 2M urea wash stage as in Example 4, were placed directly in SDS loading buffer for preparative SDS-PAGE in 10% SDS-polyacrylamide gels. The proteins were visualised by staining with 0.05% Coomassie Blue for 10 min. The gel segments were cut and homogenized in sterile PBS, mixed with Freund's adjuvant and injected directly into white rabbits intramuscularly and subcutaneously on the first, sixth and twenty first days with about 200-500 μg of fusion protein per injected dose. The rabbits were bled 14 days after the last booster dose. For immunisation of mice, 12-day old female Swiss mice were immunised with the soluble proteins of E, NS1, NS2, NS3 and NS5 fusion proteins with or without Freund's adjuvant. The injections were intraperitoneal or subcutaneous on the first, fourth, and fourteenth day, using about 20 μg fusion protein per dose. The mice were bled 14 days after the last dose. The sera of rabbits and mice were used for IFA and immunoprecipitation assays.

EXAMPLE 7

**RADIOIMMUNOPRECIPITATIONS**

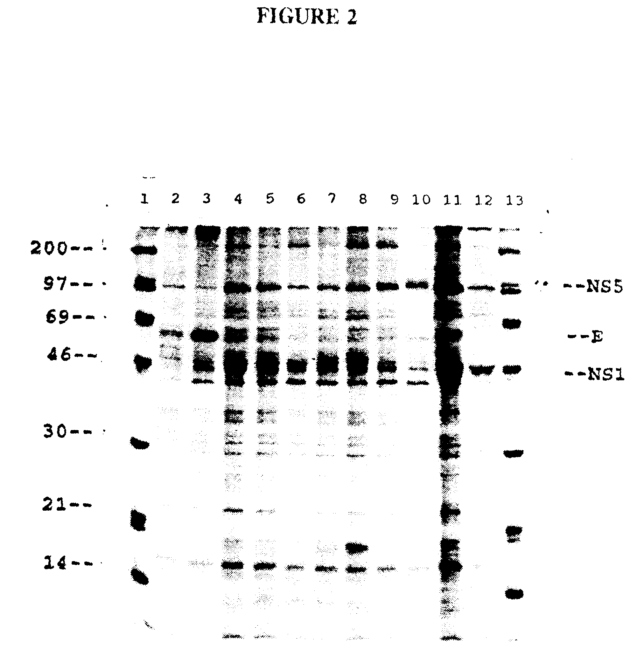

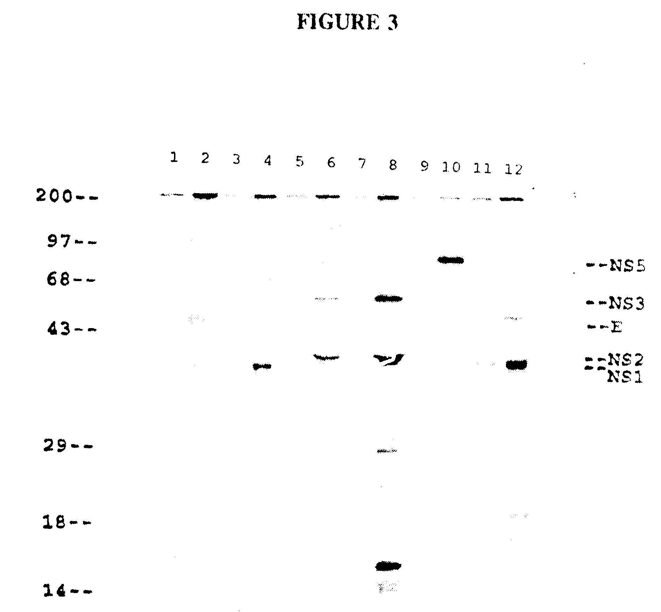

Radioimmunoprecipitations were done with rabbit and mouse antibodies against the structural and non-structural Dengue virus recombinant fusion proteins of D-275. At 36-40 h post-infection of C6/36 cells with Dengue virus S275/90 strain, cell culture medium was replaced with methionine-free medium containing 3 μg/ml actinomycin D for 3 h, followed by the addition of fresh medium with [$^{35}$S] methionine at 20 μCi/ml and 3 μg/ml actinomycin D for a further 3 h. The cells were washed with cold PBS, dissolved in RIPA buffer [100 mM Tris-HCl pH7.5, 150 mM NaCl, 10 mM EDTA, 0.1% SDS, 0.1% NP 40, 1% sodium dexoycholate, 100 μg/ml PMSF] on ice for 1 h, then clarified at 1000 x g for 10 min. The lysates were precleared with normal serum and protein A Sepharose. For immunoprecipitation, rabbit and mouse sera that had been preabsorbed with normal, uninfected C6/36 cell extract fixed by cold acetone were incubated with labeled antigen overnight at 4°C. The virus protein-antibody complexes were precipitated with protein A-Sepharose and were washed with immunoprecipitation buffer [10 mM Tris-HCl, pH7.4, 0.05% aprotinin, 1% NP40, 2 mM EDTA, 0.15 M NaCl], 6 times then 2X SDS-PAGE buffer was added, boiled for 2 min, and the supernatant was loaded on a 12% SDS-polyacrylamide gel. After fixing enhancing and drying, the gel was exposed to X-ray film. The results confirmed that antibodies to recombinant E, NS1, NS2, NS3 and NS5 had been generated in mice (Fig. 2) and in rabbits (Fig. 3). These antibodies reacted with the native E, NS1, NS2, NS3 and NS5 proteins synthesised in infected C6/36 cells.

EXAMPLE 8

**INDIRECT IMMUNOFLUORESCENCE ASSAY**

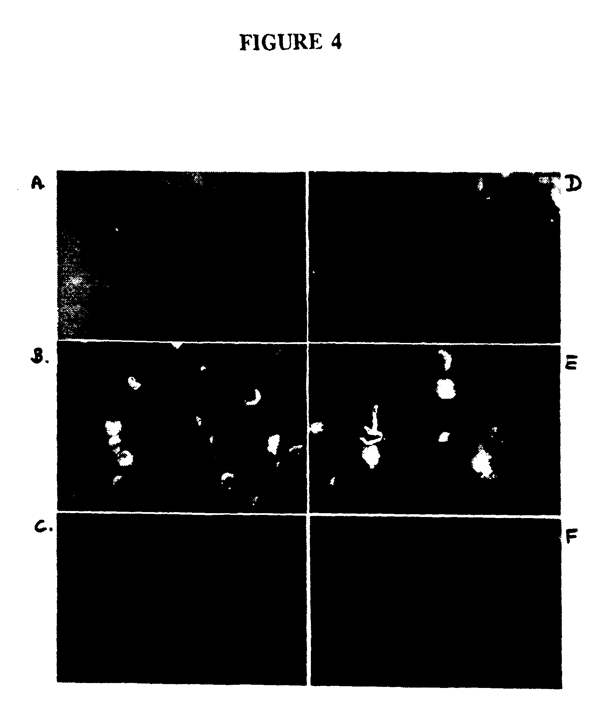

The C6/36 cells infected with Dengue virus S275/90 for 2 days were fixed on glass plates with cold acetone for immunofluorescence. 2-fold dilutions of the sera of rabbits or mice were incubated with the fixed cells for 1 h at 37°C, then washed with PBS. Secondary antibodies were linked to fluorescein and incubated for 1 h, followed by washing with PBS for observation using fluorescence microscopy. Fig 4 shows the antisera to E, NS1, NS2, NS3 and NS5 reacted specifically with the Dengue virus S275/90 infected cells, but control antiserum did no react. Quantitation of the result (as set out in Table 4) showed that an immune response to all recombinant Dengue virus proteins (E, NS1, NS2, NS3 and NS5) occurred in both mice and rabbits.

## TABLE 3

### Oligonucleotides used to prepare cDNA fragments corresponding to Dengue virus proteins (by PCR)

1.  pGEX-KG/EX-20

    DIF920E EcoRI          E

    5'CCA TGA ATT CCC ATG CGA TGC GTG GGA

    DIF2400X  Xhol          E

    5'CAC ATC TCG AGT CCG CTT GAA CCA TGA

2. pMAL-c/NS1-104

    DIR2400S          SmaI——NS1

    5' TGG TTC CCG GGG ACT CGG GAT GTG TA

    DIF3458H  HindIII      NS1

    5'ACT AAG CTT GAT CAT GCA GAG ACC ATT GA

3. pMAL-cRI/NS2-1

    DIR-NS2PM _ EcoRI          NS2

    5'AAT CAG AAT TCT CTG CAG GGT CAG GGG AA

    DIF-NS2H  HindIII  _NS2

    5'ATA ACA AAG CTT ATC TTT GTT TCT TTT TCT

4. pGEX-KG/NS3 BHC6001

    DIR-NS3B BamHI    _NS3

    5'GAA AGG ATC CTC TGG AGT GTT ATG GGA CAC A

    DIF-6360H_HindIII   NS3

    5'ACC CAA GCT TCA TCT TCT TCC TGC TGC

5. pGEX-KG/NS5(C600 HF1)

    DIR-75445 SalI          NS5

    5'AGG AGG TCG ACG AGG TAC GGG AGC C

    DIF-8365

    5'CAA TGA TAT CTA GGT TGG CT

TABLE 4

| IMMUNE RESPONSES OF MICE AND RABBITS: INDIRECT IMMUNOFLUORESCENCE ASSAYS | | |
|---|---|---|
| Dengue virus type 1 recombinant proteins | No. of mice | $\bar{\Sigma}$ Titrations of IFA |
| E | 11 | 14.91 |

TABLE 4   (continued)

| IMMUNE RESPONSES OF MICE AND RABBITS: INDIRECT IMMUNOFLUORESCENCE ASSAYS | | |
|---|---|---|
| Dengue virus type 1 recombinant proteins | No. of mice | $\bar{\Sigma}$ Titrations of IFA |
| E + CFA | 10 | 39.62 |
| NS1 | 10 | 14.89 |
| NS2 | 10 | 12.05 |
| NS2 + CFA | 10 | 12.07 |
| NS3 | 11 | 10.94 |
| NS3 + CFA | 10 | 42.56 |
| NS5 | 10 | 7.94 |
| NS5 + CFA | 10 | 10.47 |
| E + NS1 | 17 | 16.66 |
| NS3 + NS1 | 18 | 10.87 |
| NS2 + NS3 | 14 | 9.23 |
| NS5 + NS3 | 10 | 32.14 |
| MBP | 4 | < 4 |
| GST | 4 | < 4 |
| PBS | 2 | < 4 |
| Dengue virus type 1 recombinant proteins | No. of rabbits | $\bar{\Sigma}$ Titrations of IFA |
| E | 1 | 160 |
| NS1 | 1 | 160 |
| NS2 (67) | 1 | 2560 |
| NS2 (68) | 1 | 640 |
| NS3 | 1 | 2560 |
| NS5 | 1 | 160 |

## Claims

1. DEN1-S275/90 (ECACC V92042111)

2. DEN1-S275/90 (ECACC V92042111) in inactivated form.

3. A DNA polynucleotide encoding DEN1-S275/90 (ECACC V92042111) whose sequence is substantially as shown in Sequence ID No. 1.

4. A fragment of a DNA polynucleotide as claimed in claim 3, said fragment encoding one or more polypeptides selected from the C, C', PreM, M, E, NS1, NS2A, NS2B, NS3, NS4A, NS4B and NS5 polypeptides of DEN1-S275/90 (ECACC V92042111).

5. A DNA polynucleotide or a fragment thereof according to claim 3 or claim 4 in an expression vector.

6. An expression vector as claimed in claim 5 which is a plasmid or viral vector.

7. A cell harbouring an expression vector as claimed in claim 5 or claim 6.

8. A cell as claimed in claim 7 which is <u>E. coli,</u> a yeast cell or an insect cell.

9. A polypeptide in substantially isolated form which is the C, C', PreM, M, E, NS1, NS2A, NS2B, NS3, NS4A, NS4B, or NS5 polypeptide of DEN1-S275/90 (ECACC V92042111) as identified in Table 1 with reference to Sequence ID No.2

10. A polypeptide as claimed in claim 9 which is in the form of a fusion protein.

11. A method of preparing a polypeptide as claimed in claim 9 or claim 10 which comprises culturing a cell line according to claim 7 or claim 8 and recovering the polypeptide.

12. A polypeptide as claimed in claim 9 carrying a label.

13. A vaccine comprising one or more polypeptides as claimed in claim 9 or claim 10 or the inactivated virus as claimed in claim 2 in combination with a pharmaceutically acceptable carrier or diluent.

14. A vaccine as claimed in claim 13 wherein one polypeptide is selected from E, NS1, NS2, NS3, NS5 and fusion proteins thereof capable of eliciting antibodies to a DEN1 viral protein.

15. A method of preparing antibodies capable of binding a DEN1 viral protein wherein immunisation is carried out employing as the immunogen one or more polypeptides as claimed in claim 9 or claim 10 or the inactivated virus as claimed in claim 2.

16. A method as claimed in claim 15 which further comprises labelling one or more antibodies capable of binding a DEN1 viral protein.

17. A test kit for the detection of the presence or absence of DEN1 virus comprising a polypeptide as claimed in claim 9 or claim 12 fixed to a solid support.


**Patentansprüche**

1. DEN1-S275/90 (ECACC V92042111)

2. DEN1-S275/90 (ECACC V92042111) in inaktivierter Form.

3. DNA-Polynukleotid, das für DEN1-S275/90 (ECACC V92042111) codiert, dessen Sequenz im wesentlichen wie in Sequenz ID Nr. 1 gezeigt ist.

4. Fragment eines DNA-Polynukleotid nach Anspruch 3, welches Fragment für ein oder mehrere Polypeptide codiert, gewählt aus den C-, C'-, PreM-, M-, E-, NS1-, NS2A-, NS2B-, NS3-, NS4A-, NS4B- und NS5-Polypeptiden von DEN1-S275/90 (ECACC V92042111).

5. DNA-Polynukleotid oder ein Fragment davon nach Anspruch 3 oder Anspruch 4 in einem Expressionsvektor.

6. Expressionsvektor nach Anspruch 5, welcher ein Plasmidvektor oder viraler Vektor ist.

7. Zelle, welche einen Expressionsvektor nach Anspruch 5 oder Anspruch 6 beherbergt.

8. Zelle nach Anspruch 7, welche E. coli, eine Hefezelle oder eine Insektenzelle ist.

9. Polypeptid in im wesentlichen isolierter Form, welches das C-, C'-, PreM-, M-, E-, NS1-, NS2A-, NS2B-, NS3-, NS4A-, NS4B- oder NS5-Polypeptid von DEN1-S275/90 (ECACC V92042111) ist, wie in Tabelle 1 bezüglich der Sequenz ID Nr. 2 identifiziert.

10. Polypeptid nach Anspruch 9, welches in Form eines Fusionsproteins vorliegt.

11. Verfahren zur Herstellung eines Polypeptids nach Anspruch 9 oder Anspruch 10, welches das Züchten einer Zellinie nach Anspruch 7 oder Anspruch 8 und das Rückgewinnen des Polypeptids umfaßt.

12. Polypeptid nach Anspruch 9, welches eine Markierung trägt.

13. Impfstoff, umfassend ein oder mehrere Polypeptide nach Anspruch 9 oder Anspruch 10 oder den inaktivierten Virus nach Anspruch 2 in Kombination mit einer pharmazeutisch geeigneten Trägersubstanz oder Verdünnungsmittel.

14. Impfstoff nach Anspruch 13, wobei ein Polypeptid gewählt wird aus E, NS1, NS2, NS3, NS5 und deren Fusionsproteinen, der zur Hervorrufung von Antikörpern gegen ein DEN1-Virusprotein in der Lage ist.

15. Verfahren zur Herstellung von Antikörpern, die zum Binden eines DEN1-Virusproteins in der Lage sind, wobei die Immunisierung durchgeführt wird, indem als das Immunogen ein oder mehrere Polypeptide nach Anspruch 9 oder Anspruch 10 oder der inaktivierte Virus nach Anspruch 2 verwendet wird.

16. Verfahren nach Anspruch 15, welches außerdem das Markieren eines oder mehrerer Antikörpern umfaßt, die zum Binden eines DEN1-Virusproteins in der Lage sind.

17. Analysesatz zum Nachweisen des Vorhandenseins oder der Abwesenheit des DEN1-Virus, umfassend ein Polypeptid nach Anspruch 9 oder Anspruch 12, das auf einem festen Trägermaterial fixiert ist.

**Revendications**

1. DEN1-S275/90 (ECACC V92042111).

2. DEN1-S275/90 (ECACC V92042111) sous forme inactivée.

3. Polynucléotide d'ADN codant pour DEN1-S275/90 (ECACC V92042111) dont la séquence est substantiellement telle que présentée dans Séquence ID No. 1.

4. Fragment d'un polynucléotide d'ADN tel que revendiqué à la revendication 3, ledit fragment codant pour un ou plusieurs polypeptides choisi dans le groupe constitué par les polypeptides C, C', PreM, M, E, NS1, NS2A, NS2B, NS3, NS4A, NS4B et NS5 de DEN1-S275/90 (ECACC V92042111).

5. Polynucléotide d'ADN ou un fragment de celui-ci selon la revendication 3 ou 4 dans un vecteur d'expression.

6. Vecteur d'expression tel que revendiqué à la revendication 5 qui est un plasmide ou un vecteur viral.

7. Cellule hébergeant un vecteur d'expression tel que revendiqué à la revendication 5 ou à la revendication 6.

8. Cellule telle que revendiquée à la revendication 7 qui est E. coli, une cellule de levure ou une cellule d'insecte.

9. Polypeptide sous forme sensiblement isolée qui est le polypeptide C, C', PreM, M, E, NS1, NS2A, NS2B, NS3, NS4A, NS4B ou NS5 de DEN1-S275/90 (ECACC V92042111) tel qu'identifié dans le tableau 1 en référence à Séquence ID No. 2.

10. Polypeptide tel que revendiqué à la revendication 9 qui est sous la forme d'une protéine de fusion.

11. Méthode de préparation d'un polypeptide tel que revendiqué à la revendication 9 ou à la revendication 10 qui comprend les étapes consistant à cultiver une lignée cellulaire selon la revendication 7 ou la revendication 8 et à récupérer le polypeptide.

12. Polypeptide tel que revendiqué à la revendication 9 portant un marqueur.

13. Vaccin comprenant un ou plusieurs polypeptides tels que revendiqués à la revendication 9 ou à la revendication 10 ou le virus inactivé tel que revendiqué à la revendication 2 en combinaison avec un diluant ou un véhicule pharmaceutiquement acceptable.

14. Vaccin tel que revendiqué à la revendication 13 dans lequel un polypeptide est choisi dans le groupe constitué par E, NS1, NS2, NS3, NS5 et des protéines de fusion de ceux-ci capables d'induire des anticorps contre une protéine virale de DEN1.

15. Méthode de préparation d'anticorps capables de se lier à une protéine virale de DEN1 dans laquelle l'immunisation est effectuée en employant en tant qu'immunogène un ou plusieurs polypeptides tels que revendiqués à la revendication 9 ou à la revendication 10 ou le virus inactivé tel que revendiqué à la revendication 2.

16. Méthode telle que revendiquée à la revendication 15 qui comprend en outre le marquage d'un ou plusieurs anticorps capables de se lier à une protéine virale de DEN1.

17. Kit de test pour la détection de la présence ou de l'absence de virus DEN1 comprenant un polypeptide tel que revendiqué à la revendication 9 ou à la revendication 12 fixé sur un support solide.

prm     E     NS1     NS2     NS3     (NS4)     NS5

pGEX-KG/EX-20

pMAL-c/NS1-104

pMAL-cRI/NS2-1

pGEX-KG/NS3 BH c600-1

pGEX-KG/NS5 c600 HF1

FIGURE 1

EP 0 638 122 B1

# FIGURE 2

# FIGURE 3

## FIGURE 4